# EUROPEAN PATENT APPLICATION

(11) **EP 2 388 321 A1**
(43) Date of publication of application: **23.11.2011**
(21) Application number: 10731241.5
(22) Date of filing: 13.01.2010
(51) Int. Cl.: C12N 15/09, C12N 1/15, C12N 1/19, C12N 1/21, C12N 5/10, C12P 7/40, C12P 21/02, C12N 9/78

(54) **NOVEL EXPRESSION VECTOR**

(30) Priority: 13.01.2009 JP 2009004905
(71) Applicant: Daicel Chemical Industries, Ltd., Osaka 530-0001 (JP)
(72) Inventor: MATSUMURA, Eitaro, Himeji-shi Hyogo 671-1283 (JP); HAMASAKI, Momoko, Himeji-shi Hyogo 671-1283 (JP); MATSUYAMA, Akinobu, Himeji-shi Hyogo 671-1283 (JP)
(74) Representative: Grünecker, Kinkeldey, Stockmair & Schwanhäusser
(86) International application number: PCT/JP2010/050245
(87) International publication number: WO 2010/082572

(57) **Abstract**

The present inventors constructed a transformation system which uses as a host the *Kocuria rhizophila* NBRC 103217 strain which can maintain its cellular structure in various organic solvents and shows little protein leakage.

As a result, by developing novel shuttle vectors derived from *Kocuria varians* NBRC 15358, the present inventors successfully induced the expression of a foreign protein in microorganisms belonging to the genus *Kocuria* which can maintain their cellular structure in non-aqueous systems and show little protein leakage.

SEQUENCE LISTING

<110>  DAICEL CHEMICAL INDUSTRIES, LTD.

<120>  Novel expression vector

<130>  D1-A0806P

<150>  JP 2009-004905
<151>  2009-01-13

<160>  25

<170>  PatentIn version 3.4

<210>  1
<211>  809
<212>  DNA
<213>  Kocuria varians NBRC15358

<400>  1
tctagagcgg tctgcgggcc acctacaacg gggtggcccg cagaccgcgt gtctggcgac      60

acgccctgga catgcggaag ggcaccgcgc caacggtgcc ctatcctgaa gttgaactga     120

aacagtgagt ccattctagc gcctgcgcca acgggtgcca atggagcgcc cagcgatgga     180

agacctgggt ggggcctgag acgcggcccc gtatcaacat caccagccgc cttacacgcg     240

gtgctccccg ggtcagagcc ggttcctttt gccgagcgca cagacggggg ccgtcaccag     300

ggatcgcaag acctggatag cgaaggagtg ccaagcccta cagtgccgct cgtggacagc     360

agccctgcca cgcagcgagc accggagctg tgcgcacatg ccgccactgc ccacgagtaa     420

tgccgtgagc cttcttcaac cacccatgag gtggtcaacc gatgcgaccg atcgaccgac     480

cgggctgatc cgcaccgtgt tccaagatgc cttagacgtt ccccacccca cccttcttca     540

ccgaaggtgt gccccgggaa cgtttaaggc atccccctgc cctccgctcc ctccctccct     600

cccaggctgg ccggttggtc cactcctgaa cagcaccaca gaacggactg gggaagagcg     660

gagggcagga aaagaatgag cgcgagcgaa gcgagctcct ttccccgcca cagctagaac     720

accaggccct ttgggctggt cggtgaccga ggaagaacct cgttcacgca tcccaccttg     780

ggtaggtggg ggatcgtctt ccgctgcgc                                       809


<210>  2
<211>  20
<212>  DNA
<213>  Kocuria varians NBRC15358

<400>  2
tctagcaagg aggccaacat                                                  20


<210>  3

<211>  30
<212>  DNA
<213>  Kocuria varians NBRC15358

<400>  3
ttgacctgcg gaacggcggc gatggacagt                                        30


<210>  4
<211>  138
<212>  DNA
<213>  Kocuria varians NBRC15358

<400>  4
gtcgaccgag ggtagggcgg gagtgggcgc ggggaacgtg gtgtgacgga acgtgaccgg      60

ggcccgggag tcgtacgcca gtggctgaac atccttgacc tgcggaacgg cggcgatgga     120

cagttgaacc atcacgcc                                                   138


<210>  5
<211>  10913
<212>  DNA
<213>  Kocuria varians NBRC15358

<400>  5
gaattccttg cattcaagag gacagaagcc aaaacgcgga cagggaatag cacgctacag      60

gtgactcgga tactgttctg ccccaactga gcaatacatt ataatggttt tgatgaaaaa     120

ttacattcgc caaatccccg tggatggtcg gacgccgccg atgagcgatg aggagttcgc     180

tgctgttgtt gcccaggcag gcgatcccgc catgttcggg gattggtaca ccaaaagcgt     240

gaaatcctcc aatggtgcag gagaaggtga gtttactggc cgcatctcta ttgacttgtc     300

tgattatctg aatgcgttct ttcccgggga aattgaaaaa cgcatcaagg cggttcagga     360

gcagcttccg catgttcccg attccgaaac ggtaaggaaa ctagtcacag acgcactcac     420

cccggacggg tggtcttctt tggtgcggcc gttctctata gcgcgcatcc ctgcctggag     480

cacgtattta tatcgggtgc gccctggact tcacggccct gaagacatca agactgtcgg     540

ggacatctgg acaccaccca ctggcacctc gccaggacgc gtcaaccgtc gaggtcaacc     600

catcctttac acctcagcgc aatacccctc cgtcgctttc cgcgaatcga gagccaaggt     660

tggagaagtc gtcgcgctta gctgcttcgc cataactgac gatctcaacc tgctcaacct     720

tgacgatatg agtccgcagg ggaacttgaa caccagtcag caacgcaaat ggcggcagct     780

tgcaaaattt taccggtggg ccttccggga ttcagggagc gacttgaata gtccacaaca     840

tcttgtaccc caagtattga ctctggactt aaatatgctc atcccgcccc tcgtcggtta     900

tggttatcaa tccgtcattg tcaaccacca cgccgcgatc aacgtggccc tagacgagca     960

aaaagcaatg gatcacctac gcctggtagg cacgactgtg tggcgtattg gcaagcccac    1020

aagcatgctt cttgcctcca tggtaccgaa acatccgaga cttactagag acacgccgct 1080

ggtcccaggt aactttgcat ggcctccaga tcttgaagtc acccaaaggt cgtatcagag 1140

ccgcgcatag ccagcgtctg gccatcgcac ctcaccccat atataaatgg ggaggccaga 1200

tgtagtaccg cggttcctga tggcccagcc gtttttaccg agcaacccgc tcgtctgatc 1260

ctatgaggcg ccgacgaact cagacggtcg ctgtggaggg cagatttcgg atccgccggt 1320

agagagttgc cctagatatc ccgagatctc gagctacctg ggcgactgga tccccggact 1380

cgattaatcg gacggcagtg cggatctgag aatcagtgaa agtttggcga cgtcccccga 1440

gatccttgcc agcggcccta cgtttctcca ccgagtcagt gatccgctca cgcttaatct 1500

ccaactccat ctgtgccaac gcggccatca cggtgaacac catcgacccc atcgggatcg 1560

atgtgtccac gtctcccccg ccgaggttga gcacccgcaa ccccacgccg cgctgccgaa 1620

gttcttgagc cagttccagc atggtctgag tggagcgccc caaccggtcc aaggtcgtca 1680

ccaccagggt gtcgccctcg tggagagctc ctacggcttg gtcaaagccc gggcgggatg 1740

cccgggcccc cgagaccccg tgatccacgt agaggtcatc ccgacgcacc cccgcgccca 1800

gcaggtctgt cacctgccgg tccgtgccct ggctcctcgt agacacccgc gcgtacccaa 1860

tcaacttccc catgctctgt ctccgcctcc cgtatgtctc gcaaccgacc gtcagcaccc 1920

cgatcggggc gaccggttga gagacaacgc tacgagacag cccccgcccc ggatctccgg 1980

cgccagtcca agacgccatg agacgtcttg caacccattg attgcgagac tcattccgtc 2040

gcgtctcgcg tctctcgcgc ttggcacagg ggacttggac accgtgtacc actttgcttt 2100

gcctgagctc atcgaggcgg tcgcggcgac gggcaacgat gaggcgcaga ccatgctcac 2160

catgctgctc agcggtaagc gtctacgcga cattagcgac ttgccgctcg acctggcggt 2220

ctagcggcaa gaatcagacg ccgcaacact tcatggcctg agatcgccgc gggggctggt 2280

gcgccgatcg ccgaccccag gtatcaaaac gcttgaagca ggggggtttc atgccacccc 2340

ctgcaggggt gtcagaatag gtgtcatgac cacctatggt gacaccccca acaacaaggt 2400

ccctagccag ggaggggtat cagggggttc cagtgcggtg ttcaccatga aggaagccgc 2460

cgatgtggca ggtgtcagcg tgagcaccct ccgccgacgg cgcgccgagc tggaaggagc 2520

aggggccacc atcacctccg ccgggtggca ggtgcccatg accgcactca tcgccgtggg 2580

cctcatccac ggcgaaggtt tccataccgc cccccaggcc agaccatcga cgccgtctgc 2640

ccagtccccg gatgaagcaa agagcaccgg gcgtctgctc gagcgcgtcc gtgagttgga 2700

agctgaggtc caggaatggc gacggcgcgc cgaagttgct gaagcacgag ccgaagaacg 2760

ccgccgagct ctcgatgccc tccagatcgc caacgaaacc gaacgcatag cgctgcggat 2820

gctcaccggg cagagctccc acacaccaca ggctcctacg gctcccgagc ctgctcgtgc 2880

caggccggcc gaggagccgg aagcatcccc gggacccacc acacccgtga cctctcaaga 2940

gccgcgccgc gggttctttt cccgcatctt ctcggcctaa gagcttccgc cgataggtgt 3000

ggtggagcgg tggcatgaga caggcacgag acctggtaaa cacggatttg ttgagaatca 3060

gtgtgttgac gaggagcact cgtgcctgcc gttccatcgt gcatcatcga acccatctgg 3120

gaccagttcc agatgctgat ccctgctgtg gcggacgagc atccattggg ttgccaccgg 3180

ccacggatcc cggaccgggt ggtgttcgac aagctggtcc aggtcctcgt ccccggggcc 3240

gcctacgaga ggatctccga cacgacgtgt tcggctacca cgatccgccg ccggcgtgat 3300

gagtggatcg aggccggagc ctttaaggag ctggaacagt tttgtctgga gggctacgag 3360

aagatggtcg ggctccagct ggagaacttg gccgtggacg ggtgcatcac caaggccccg 3420

tgcggtggcg aggccgccgg gcggtcaccg gtggaccgcg gcaagcaggg cacgaaacgg 3480

tcgctgctgg tcgagggcgc cgggatcccg ctgggtaccg tggtggcccc ggccaaccgc 3540

cacgactccc cgttgctgcg cccgacgttg gagacgctgt ccaggttcgg tttcgaccgg 3600

ccggagcgga tcaccgtgca cctggacgcc gactacgact cgaagggcac ccgggggttg 3660

ctcgccgagc tcggctgtga agccaggatc gcggtcaagg gcgtgcccgc cccgatccag 3720

aacacgtcca ggtgggtggt ggaacggacg aactcctggc acaaccgcgg gttccagaag 3780

ctggccgtct gcaccgagaa acgtatccgg gtaatcgagg cgttcatcgc cctggccaac 3840

gccatcatca tcactcgcag gctcctccgc gaggcctgga acacccaccg ctgggccacc 3900

cgcccgaccc gacgaccatg gcctaatggc ggaacctctt actcatcctc cacggccagg 3960

cgcaggtggt catcgcccta cacggatacc acaaggcttt gccgcatgtt ggcaatggcc 4020

aggcgccggt aaccagtcgc gagaagatcg tcgcttccca cggcggacag cgcactttta 4080

gacacctcct gcgttcgtca aaagaagcgg tccgggatca ttcccggttt cagcccggac 4140

cgccgcgatc cgtcgacgac gtcgagtgct tcaccctggc ctggatcgac aggtacaaca 4200

gcgcccgccc gcacacccgc ctcggcaacg ccccacccgc cgagtacgag gcagcgcact 4260

gcgctgagcc caccaagtca tcgcgaccgg caatgacacc tgcatagacg tggcatcaac 4320

gcgtagatgt ttcagtttct tctgtgctgg ggctgtgtca ggtgctggac cgggtgtagg 4380

cagcggcgcg caccacatct tcgtcctcca gtccagctcc tagtgctccg cccatggtgg 4440

ccaggctggc ggtgagccag ctgatcttcg cgtagtccaa agcgttcact gggtgaccgg 4500

cgacaccggc cagtacctgc tcgtcgatga gcagcagcgc gccgacccag gacaggatga 4560

ataagacgag gtagaacacc acaaccccga tggccacggt cgcggtggtg gccaaattga 4620

acaggatcac ctgctcacgt tgggctcgcc gccgtgggcg ctcccacaag tcggctccca 4680

ggatcagggt ggcactcacc gccccgatcg ccatcaccgc cagcaggacc aggcgcaccg 4740

gcccgtaggc catggccagt acccataggt ccgaggccac caatgtcagc atgccggtgg 4800

ccgctgccac gaccagcgcc cgggagagcc gggccacgaa gacccaaggc tggttggcgc 4860

ggatcatgcc cagcagcaac cgcacgttgc cgctcagcac tcgggccgtg aactgcacga 4920

cattgtcgtc cgggcgttcg tcgagatcgg tggccagctg gtgggctctt cgagcaagat 4980

cccgctgggc gttgaggtct tcggcgtcgt agcccaggag ctgcccgacc acgtgcacgg 5040

tggtctcttg caccttctgc cgcacgcgca cggctcccag ggttgggacg gagaccagtc 5100

ctaccccgtg cacggggctg agctgggtga gcaccggacg tcggccggtc ttcaggggga 5160

tctcggtgag caccacggcc aaatcccagt tctcctccag cacgcggttt cgggccgcct 5220

ccagcaggtc caaggtctcg gtgggtggat cgaccagccg gtcttcggcc atcttcagtt 5280

gccagcgcac tcccggatac cgctgcacca aaatcttgcg caccgaggtg gtgacctcgg 5340

ggccgagcac cgcactcagg gatggtgaga ccaccaggcc gatgagcagc tcggcatcgg 5400

aaaggggttc ggcaggatcg gcggtggatc ggcaacgctc agcagtcacc gctccatagt 5460

gaggcaacca cgggccggat tgccactggc tcgatggagc ccccaaggtt tcgtgcagca 5520

ccgcgggccg gattcggtgg tcgcctcgat cccggaaaga gcggtgctgc tcactgcgaa 5580

atcctctccc cgccgacgcc gccagcgcga agacatcaca gccagcgggc ccgggacctg 5640

ccggtgtcga ggacggcttc tcgatgatgc tgcagtgacg gatcggttga agtgcttcgg 5700

cggtgtggga gcagtgatca gcgtggggcc ggtcgattcc gttcggacag gtgatgcatc 5760

gactgcccag cggcaggtgc ggtactgatg cgggggtacc cggtgcaggg gtgtcaaggc 5820

ccagctccga agacggcaag ccgggcacgt gaaaggcgaa ttatggcccg ccgcaacccg 5880

agcgggaagg catgtgtggc acgaggcgcc gaagacgggc cgggcgttta ctgcagagat 5940

aaatatgctt atcctggtaa ggtccgcgac atcttgtaag gccctgagag cgggcccgtc 6000

gctggtccac cacggaccga cgggctcggg gacggggctg aggacccgga gaggagccag 6060

tggcagaagt ccacgaagat cgcaggcacc gcggctgaac ggccgatacc gagggggagc 6120

aagggaggcc agggctgctg atgtgcccgg actgttccgc gatttctctt cctaactttc 6180

cgtccactcc cgacccaggt gcccggcaac cccgggtgca ggccaccgtc cgggtcggat 6240

cgtcaccacg aacaggaaac gataagcatg tcccatccct cccctcttcc acccactgct 6300

tcgactcatt accaggtcct gcgcacagtg ccctcctatg accaggctca gcgccttgtc 6360

gatcgactct ccgacgccgg gttcccggtt gagcacgtgc gcgtggtcgg caccgatctg 6420

cgcctggttg agcaggtcac cgggcggatg acctacggca aggccgccct gtacggagcc 6480

gcctccgggg cctggctggg attgctcatt ggcttgctgt tgggtctttt cacggtcgtg 6540

gggtggctgt cggtaatcct gtgggcggta ctcctgggcg cggtgtgggg gctgatcttc 6600

gggttgctgg gccacgcggc caccggcggg cgccgggact tcagtagcgt ccagggcctg 6660

gaggcgtcct cctacgagat tctcgtggag gccgagtacc tcgaggcggc cagggccaag 6720

ctcgacggcc agccctgaca gcaccgggca cgtggacgag aacgccccca ccggcgatac 6780

cgggcccact ccataaccgc accccggcac cgaagagccc gccatcttcg acacgccccg 6840

acggggcggg ggtagcccca ccacctgccc ccgccccgcc ctgggctgct ggtgaatcgg 6900

atccgccggg cccgcatcac cgcccgggtc gaagggtgcg agtaggcctt gtcggccagc 6960

accgcgtccg ccgtgacggg ccgggccggc ccgtcacggc ggacgctgat ctgctccagc 7020

agcggcagca actgtgggtt gtccccggtc tgtccctcgg tcaggatgat gctgatcggc 7080

atcccggctc cgtcgacggc caggtggatc ttggaggtca gtcctccgcg ggaccgtccg 7140

agacattctc cggccacagc gatggcctcg accggatcgg agcagccccc tttttccggg 7200

ccccggcggc atgctggtgg gcccggatgg aggacgagtc cacggagaac acccattcga 7260

cctccccggc agcgtcgtcc ttgaccaccg cctcatccag gatcttggcc ccagtgccgt 7320

ccatggtcca gatccgcagc cgctcgtggc cggtcttcca cggcccatac cgttcgggca 7380

cgtcccgcca gggcgccccg gtgcgcagct tccacaggat cccgttgacc acctgccggt 7440

gatcccgcca ccgccggccc ttaccgttgg ccggcagcaa cggcgcgatg cgttcccagg 7500

ccttgtccgt cagttctcca cgcctcacca tgccccagga ttaacaggac ctgacgggga 7560

ttaacaaccc cacgccgccg ttccggggta cttagaggtt gcgcgggtag gtcagggtct 7620

tcggcggggg cgtgtgtccc agcggtgagt ggtccagacg gtccggatca ggcgtcgaac 7680

gatgatgatg gcgttggcca gcgcgatcca ggcgcgctgg atcgcttcgc tgcgatcggt 7740

gacgatctgc agggcacgaa agccgcgggt gtgccaggag ttcgtgcgtt cgaccttcca 7800

gcggtgggtg tggttgaccg gaacgaactg acccttcggg ttgatctgcc aggtgcagcc 7860

gagctcatcg agtagctctc gagtgacggc ggagtcgtag ccggagtcga ggtggaccgt 7920

gatggtctca ggcaggtcga acccgaacct gctcagccgc tccaggatcg ggcgcagcag 7980

cggtgagtcg tgccgattgg caccggcaag ctcgcatccg atcgggacgc cggcgccctc 8040

gacgagcagc gaacgtttca ggcccgactt cgcccggtcg accggggact tgccagtgtt 8100

gtccccgctg cacggggcct tgacgatgca gccgtcgacg accaggtcac tcaggtccag 8160

tcccacgacc gtgtcgtaag cctccagcgc ggcctgttcg agccggtcga acacccccgc 8220

ggcgatccac tcgtcgcggc gggcccgcag cagggttgtg gccgagaccc ggtcgtcggc 8280

gtgcctgtgg taagagccgc ccagcacgag ccgggccacg agcttgtcga acaccacccg 8340

gtccggtatc cgccgccggt gacaccccaa cggatgggtg tcgacccgcg caggaatcag 8400

ggcctcgaaa cggctcgatg aacgatgctg gcaccgcagg cacgggactc ctcgcgacga 8460

cgtagatcta ggcaatccac atcgaagcgg acatcccgtg cctgcgtcac atccccgaca 8520

agccgcgccc tacccgcgcg acctctaagc ccagctacag gccccgccca cgatcccccc 8580

ggccggggcc gggacgacga aggtgctgca cctgctccac cgaatgaggt ggggctaccc 8640

actgctgctg ttggggtgcc ggggactgtt tccggggtgc cgggttgtcc agctccacga 8700

acttgcggat ctgctcccac tccgcgcggc gggccgccac cacctgagcc tgctgctcct 8760

gctgctgacg caccgcgagc actgaacgtt tgccgcgtcc cgcctcacgg cgccgcggtg 8820

cggtctgcaa accgtgctcc aactccagac gggtgcacgc gctttgggcg gcgcggcggt 8880

cgttgcgacc gtgccacacc gtcccctcgt cactgacccg gcacaccacc acatgcacgt 8940

ggtcatcggc gtggcggacc atcacccacg ggtgctccgc gaaccccatc gactccgcaa 9000

acgtctggcc tgcctgcgcc cactccgcat ccgtcaaccg ccgatcccca gccgtgttgc 9060

gcaaagaggc ctgccagatc ggtttcgtga tctccgggcg ggtgcgcatc gccgcgcgca 9120

tctccttcac ccacgccttc tcatccgcat ccccggtcac gaacacgttc ccgcccacga 9180

ccacaccacc ggcataccgg cccccgttgc gttcgtagaa gtgctcattg gcatgccccg 9240

gcccatgcag ataagccccg atgttgcccg ggttcgcccc ccgggtgatc ttcgcgatca 9300

cggcaactgc ctccgcacta ctcccagctg actcgccagc gcgtccacgg cctcatacag 9360

ctgcggggtc gggacgacct gcccctggtt caccgcacgc atcaactggt tcagattcga 9420

ccccacccgc gccagatccg cccggatcct cgccaactcc tgcacctcac ccatccgcgg 9480

actctccgca tcaagctgtt cctggactcg ttcccgagcc cacaccgaca accgttgccc 9540

gccggcggcc tccgcccacg cccggtgctc agcctcggtc aacgacatcg acacccgccg 9600

ggtccgcggc tccaccgcca ccggccgccc accagcaccc cgcatccgag gcgccgacgc 9660

tgtatcggtt ggttccgggt ccggtgaggc gaggggctgc tcgaggagca gcccctccca 9720

ccggttcccg gaccccgacg ggaccgtgcc cggcgaggtc gaacgacgtg agaacaagcc 9780

caacggacca accctttcga gaccccgcca aggggtcgag accagccctc cgcaggagca 9840

aggggattga ccggaacggg aacacctggc agcgaggaac gagcggtgcg ccaggttttc 9900

cgttccggac aatcatacct tgcatccccg tttcagcgcc ccgacgaagg aggagaacag 9960

cgcgctggaa cagggggcgt ggtggtgtgc tgggcctgac cgacgcagga ggacagagcc 10020

agggccaggg caccaccacg catcgtctgc agcgcagcgg aagacgatcc cccacctacc 10080

caaggtggga tgcgtgaacg aggttcttcc tcggtcaccg accagcccaa agggcctggt 10140

gttctagctg tggcggggaa aggagctcgc ttcgctcgcg ctcattcttt tcctgccctc 10200

cgctcttccc cagtccgttc tgtggtgctg ttcaggagtg gaccaaccgg ccagcctggg 10260

agggagggag ggagcggagg gcagggggat gccttaaacg ttcccggggc acaccttcgg 10320

tgaagaaggg tggggtgggg aacgtctaag gcatcttgga acacggtgcg gatcagcccg 10380

gtcggtcgat cggtcgcatc ggttgaccac ctcatgggtg gttgaagaag gctcacggca 10440

ttactcgtgg gcagtggcgg catgtgcgca cagctccggt gctcgctgcg tggcagggct 10500

gctgtccacg agcggcactg tagggcttgg cactccttcg ctatccaggt cttgcgatcc 10560

ctggtgacgg cccccgtctg tgcgctcggc aaaaggaacc ggctctgacc cggggagcac 10620

cgcgtgtaag gcggctggtg atgttgatac ggggccgcgt ctcaggcccc acccaggtct 10680

tccatcgctg ggcgctccat tggcacccgt tggcgcaggc gctagaatgg actcactgtt 10740

tcagttcaac ttcaggatag ggcaccgttg gcgcggtgcc cttccgcatg tccagggcgt 10800

gtcgccagac acgcggtctg cgggccaccc cgttgtaggt ggcccgcaga ccgctctaga 10860

tactcttccc gtcactgcgt cggagtggcc gctagccctt cactgatctg gga 10913

<210> 6
<211> 4303
<212> DNA
<213> Artificial

<220>
<223> An artificially synthesized vector sequence

<400> 6
tctagagcgg tctgcgggcc acctacaacg gggtggcccg cagaccgcgt gtctggcgac 60

acgccctgga catgcggaag ggcaccgcgc caacggtgcc ctatcctgaa gttgaactga 120

aacagtgagt ccattctagc gcctgcgcca acgggtgcca atggagcgcc cagcgatgga 180

agacctgggt ggggcctgag acgcggcccc gtatcaacat caccagccgc cttacacgcg 240

gtgctccccg ggtcagagcc ggttcctttt gccgagcgca cagacggggg ccgtcaccag 300

ggatcgcaag acctggatag cgaaggagtg ccaagcccta cagtgccgct cgtggacagc 360

agccctgcca cgcagcgagc accggagctg tgcgcacatg ccgccactgc ccacgagtaa 420

tgccgtgagc cttcttcaac cacccatgag gtggtcaacc gatgcgaccg atcgaccgac 480

cgggctgatc cgcaccgtgt tccaagatgc cttagacgtt ccccacccca cccttcttca 540

ccgaaggtgt gccccgggaa cgtttaaggc atccccctgc cctccgctcc ctccctccct 600

cccaggctgg ccggttggtc cactcctgaa cagcaccaca gaacggactg gggaagagcg 660

gagggcagga aaagaatgag cgcgagcgaa gcgagctcct ttccccgcca cagctagaac 720

accaggccct ttgggctggt cggtgaccga ggaagaacct cgttcacgca tcccaccttg 780

ggtaggtggg ggatcgtctt ccgctgcgct gcagacgatg cgtggtggtg ccctggccct 840

ggctctgtcc tcctgcgtcg gtcaggccca gcacaccacc acgccccctg ttccagcgcg 900

ctgttctcct ccttcgtcgg ggcgctgaaa cggggatgca aggtatgatt gtccggaacg 960

gaaaacctgg cgcaccgctc gttcctcgct gccaggtgtt cccgttccgg tcaatcccct 1020

tgctcctgcg gagggctggt ctcgacccct tggcggggtc tcgaaagggt ggtcccgttg 1080

ggcttgttct cacgtcgttc gacctcgccg ggcacggtcc cgtcggggtc cgggaaccgg 1140

tgggaggggc tgctcctcga gcagcccctc gcctcaccgg acccggaacc aaccgataca 1200

gcgtcggcgc ctcggatgcg gggtgctggt gggcggccgg tggcggtgga gccgcggacc 1260

cggcgggtgt cgatgtcgtt gaccgaggct gagcaccggg cgtgggcgga ggccgccggc 1320

gggcaacggt tgtcggtgtg ggctcgggaa cgagtccagg aacagcttga tgcggagagt 1380

ccgcggatgg gtgaggtgca ggagttggcg aggatccggg cggatctggc gcgggtgggg 1440

tcgaatctga accagttgat gcgtgcggtg aaccaggggc aggtcgtccc gaccccgcag 1500

ctgtatgagg ccgtggacgc gctggcgagt cagctgggag tagtgcggag gcagttgccg 1560

tgatcgcgaa gatcacccgg ggggcgaacc cgggcaacat cggggcttat ctgcatgggc 1620

cggggcatgc caatgagcac ttctacgaac gcaacggggg ccggtatgcc ggtggtgtgg 1680

tcgtgggcgg gaacgtgttc gtgaccgggg atgcggatga gaaggcgtgg gtgaaggaga 1740

tgcgcgcggc gatgcgcacc cgcccggaga tcacgaaacc gatctggcag gcctctttgc 1800

gcaacacggc tggggatcgg cggttgacgg atgcggagtg ggcgcaggca ggccagacgt 1860

ttgcggagtc gatggggttc gcggagcacc cgtgggtgat ggtccgccac gccgatgacc 1920

acgtgcatgt ggtggtgtgc cgggtcagtg acgaggggac ggtgtggcac ggtcgcaacg 1980

accgccgcgc cgcccaaagc gcgtgcaccc gtctggagtt ggagcacggt ttgcagaccg 2040

caccgcggcg ccgtgaggcg ggacgcggca aacgttcagt gctcgcggtg cgtcagcagc 2100

aggagcagca ggctcaggtg gtggcggccc gccgcgcgga gtgggagcag atccgcaagt 2160

tcgtggagct ggacaacccg gcaccccgga aacagtcccc ggcaccccaa cagcagcagt 2220

gggtagcccc acctcattcg gtggagcagg tgcagcacct tcgtcgtccc ggccccggcc 2280

ggggggatcg tgggcggggc ctgtagctgg gcttagaggt cgcgcgggta gggcgcggct 2340

tgtcggggat gtgacgcagc tgtctcttat acacatctca accatcatcg atgaattgct 2400

tcgttaatac agatgtaggt gttccacagg gtagccagca gcatcctgcg atgcagatcc 2460

ggatgccatt tcattacctc tttctccgca cccgacatag atccgaagat cagcagttca 2520

acctgttgat agtacgtact aagctctcat gtttcacgta ctaagctctc atgtttaacg 2580

tactaagctc tcatgtttaa cgaactaaac cctcatggct aacgtactaa gctctcatgg 2640

ctaacgtact aagctctcat gtttcacgta ctaagctctc atgtttgaac aataaaatta 2700

atataaatca gcaacttaaa tagcctctaa ggttttaagt tttataagaa aaaaaagaat 2760

atataaggct tttaaagctt ttaaggttta acggttgtgg acaacaagcc agggatctgc 2820

catttcatta cctctttctc cgcacccgac atagatccgg aacataatgg tgcagggcgc 2880

tgacttccgc gtttccagac tttacgaaac acggaaaccg aagaccattc atgttgttgc 2940

tcaggtcgca gacgttttgc agcagcagtc gcttcacgtt cgctcgcgta tcggtgattc 3000

attctgctaa ccagtaaggc aaccccgcca gcctagccgg gtcctcaacg acaggagcac 3060

gatcatgcgc acccgtggcc aggacccaac gctgcccgag atgcgccgcg tgcggctgct 3120

ggagatggcg gacgcgatgg atatgttctg ccaagggttg gtttgcgcat tcacagggtg 3180

tctcaaaatc tctgatgtta cattgcacaa gataaaaata tatcatcatg aacaataaaa 3240

ctgtctgctt acataaacag taatacaagg ggtgttatga gccatattca acgggaaacg 3300

tcttgctcga ggccgcgatt aaattccaac atggatgctg atttatatgg gtataaatgg 3360

gctcgcgata atgtcgggca atcaggtgcg acaatctatc gattgtatgg gaagcccgat 3420

gcgccagagt tgtttctgaa acatggcaaa ggtagcgttg ccaatgatgt tacagatgag 3480

atggtcagac taaactggct gacggaattt atgcctcttc cgaccatcaa gcattttatc 3540

cgtactcctg atgatgcatg gttactcacc actgcgatcc ccggaaaaac agcattccag 3600

gtattagaag aatatcctga ttcaggtgaa aatattgttg atgcgctggc agtgttcctg 3660

cgccggttgc attcgattcc tgtttgtaat tgtcctttta acagcgatcg cgtatttcgt 3720

ctcgctcagg cgcaatcacg aatgaataac ggtttggttg atgcgagtga ttttgatgac 3780

gagcgtaatg gctggcctgt tgaacaagtc tggaaagaaa tgcataaact tttgccattc 3840

tcaccggatt cagtcgtcac tcatggtgat ttctcacttg ataaccttat ttttgacgag 3900

gggaaattaa taggttgtat tgatgttgga cgagtcggaa tcgcagaccg ataccaggat 3960

cttgccatcc tatggaactg cctcggtgag ttttctcctt cattacagaa acggcttttt 4020

caaaaatatg gtattgataa tcctgatatg aataaattgc agtttcattt gatgctcgat 4080

gagtttttct aatcagaatt ggttaattgg ttgtaacact ggcagagcat tacgctgact 4140

tgacgggacg gcggctttgt tgaataaatc gaacttttgc tgagttgaag gatcagatca 4200

cgcatcttcc cgacaacgca gaccgttccg tggcaaagca aaagttcaaa atcaccaact 4260

ggtccaccta caacaaagct ctcatcaacc gtggcgggga tcc 4303

<210> 7
<211> 4326
<212> DNA
<213> Artificial

<220>
<223> An artificially synthesized vector sequence

<400> 7
tctagcaagg aggccaacat atgtctagag cggtctgcgg gccacctaca acggggtggc 60

ccgcagaccg cgtgtctggc gacacgccct ggacatgcgg aagggcaccg cgccaacggt 120

gccctatcct gaagttgaac tgaaacagtg agtccattct agcgcctgcg ccaacgggtg 180

ccaatggagc gcccagcgat ggaagacctg ggtggggcct gagacgcggc cccgtatcaa 240

catcaccagc cgccttacac gcggtgctcc ccgggtcaga gccggttcct tttgccgagc 300

gcacagacgg gggccgtcac cagggatcgc aagacctgga tagcgaagga gtgccaagcc 360

ctacagtgcc gctcgtggac agcagccctg ccacgcagcg agcaccggag ctgtgcgcac 420

atgccgccac tgcccacgag taatgccgtg agccttcttc aaccacccat gaggtggtca 480

accgatgcga ccgatcgacc gaccgggctg atccgcaccg tgttccaaga tgccttagac 540

gttccccacc ccacccttct tcaccgaagg tgtgccccgg gaacgtttaa ggcatccccc 600

tgccctccgc tccctccctc cctcccaggc tggccggttg gtccactcct gaacagcacc 660

acagaacgga ctggggaaga gcggagggca ggaaaagaat gagcgcgagc gaagcgagct 720

cctttccccg ccacagctag aacaccaggc cctttgggct ggtcggtgac cgaggaagaa 780

cctcgttcac gcatcccacc ttgggtaggt gggggatcgt cttccgctgc gctgcagacg 840

atgcgtggtg gtgccctggc cctggctctg tcctcctgcg tcggtcaggc ccagcacacc 900

accacgcccc ctgttccagc gcgctgttct cctccttcgt cggggcgctg aaacggggat 960

gcaaggtatg attgtccgga acggaaaacc tggcgcaccg ctcgttcctc gctgccaggt 1020

gttcccgttc cggtcaatcc ccttgctcct gcggagggct ggtctcgacc ccttggcggg 1080

gtctcgaaag ggtggtcccg ttgggcttgt tctcacgtcg ttcgacctcg ccgggcacgg 1140

tcccgtcggg gtccgggaac cggtgggagg ggctgctcct cgagcagccc ctcgcctcac 1200

cggacccgga accaaccgat acagcgtcgg cgcctcggat gcggggtgct ggtgggcggc 1260

cggtggcggt ggagccgcgg acccggcggg tgtcgatgtc gttgaccgag gctgagcacc 1320

gggcgtgggc ggaggccgcc ggcgggcaac ggttgtcggt gtgggctcgg gaacgagtcc 1380

aggaacagct tgatgcggag agtccgcgga tgggtgaggt gcaggagttg gcgaggatcc 1440

gggcggatct ggcgcgggtg gggtcgaatc tgaaccagtt gatgcgtgcg gtgaaccagg 1500

ggcaggtcgt cccgaccccg cagctgtatg aggccgtgga cgcgctggcg agtcagctgg 1560

gagtagtgcg gaggcagttg ccgtgatcgc gaagatcacc cgggggcgga acccgggcaa 1620

catcggggct tatctgcatg ggccggggca tgccaatgag cacttctacg aacgcaacgg 1680

gggccggtat gccggtggtg tggtcgtggg cgggaacgtg ttcgtgaccg gggatgcgga 1740

tgagaaggcg tgggtgaagg agatgcgcgc ggcgatgcgc acccgcccgg agatcacgaa 1800

accgatctgg caggcctctt tgcgcaacac ggctggggat cggcggttga cggatgcgga 1860

gtgggcgcag gcaggccaga cgtttgcgga gtcgatgggg ttcgcggagc acccgtgggt 1920

gatggtccgc cacgccgatg accacgtgca tgtggtggtg tgccgggtca gtgacgaggg 1980

gacggtgtgg cacggtcgca acgaccgccg cgccgcccaa agcgcgtgca cccgtctgga 2040

gttggagcac ggtttgcaga ccgcaccgcg gcgccgtgag gcgggacgcg gcaaacgttc 2100

agtgctcgcg gtgcgtcagc agcaggagca gcaggctcag gtggtggcgg cccgccgcgc 2160

ggagtgggag cagatccgca agttcgtgga gctggacaac ccggcacccc ggaaacagtc 2220

cccggcaccc caacagcagc agtgggtagc cccacctcat tcggtggagc aggtgcagca 2280

ccttcgtcgt cccggccccg gccgggggga tcgtgggcgg ggcctgtagc tgggcttaga 2340

ggtcgcgcgg gtagggcgcg gcttgtcggg gatgtgacgc agctgtctct tatacacatc 2400

tcaaccatca tcgatgaatt gcttcgttaa tacagatgta ggtgttccac agggtagcca 2460

gcagcatcct gcgatgcaga tccggatgcc atttcattac ctctttctcc gcacccgaca 2520

tagatccgaa gatcagcagt tcaacctgtt gatagtacgt actaagctct catgtttcac 2580

gtactaagct ctcatgttta acgtactaag ctctcatgtt taacgaacta aaccctcatg 2640

gctaacgtac taagctctca tggctaacgt actaagctct catgtttcac gtactaagct 2700

ctcatgtttg aacaataaaa ttaatataaa tcagcaactt aaatagcctc taaggtttta 2760

agttttataa gaaaaaaaag aatatataag gcttttaaag cttttaaggt ttaacggttg 2820

tggacaacaa gccagggatc tgccatttca ttacctcttt ctccgcaccc gacatagatc 2880

cggaacataa tggtgcaggg cgctgacttc cgcgtttcca gactttacga aacacggaaa 2940

ccgaagacca ttcatgttgt tgctcaggtc gcagacgttt tgcagcagca gtcgcttcac 3000

gttcgctcgc gtatcggtga ttcattctgc taaccagtaa ggcaaccccg ccagcctagc 3060

cgggtcctca acgacaggag cacgatcatg cgcacccgtg gccaggaccc aacgctgccc 3120

gagatgcgcc gcgtgcggct gctggagatg gcggacgcga tggatatgtt ctgccaaggg 3180

ttggtttgcg cattcacagg gtgtctcaaa atctctgatg ttacattgca caagataaaa 3240

atatatcatc atgaacaata aaactgtctg cttacataaa cagtaataca aggggtgtta 3300

tgagccatat tcaacgggaa acgtcttgct cgaggccgcg attaaattcc aacatggatg 3360

ctgatttata tgggtataaa tgggctcgcg ataatgtcgg gcaatcaggt gcgacaatct 3420

atcgattgta tgggaagccc gatgcgccag agttgtttct gaaacatggc aaaggtagcg 3480

ttgccaatga tgttacagat gagatggtca gactaaactg gctgacggaa tttatgcctc 3540

ttccgaccat caagcatttt atccgtactc ctgatgatgc atggttactc accactgcga 3600

tccccggaaa aacagcattc caggtattag aagaatatcc tgattcaggt gaaaatattg 3660

ttgatgcgct ggcagtgttc ctgcgccggt tgcattcgat tcctgtttgt aattgtcctt 3720

ttaacagcga tcgcgtattt cgtctcgctc aggcgcaatc acgaatgaat aacggtttgg 3780

ttgatgcgag tgattttgat gacgagcgta atggctggcc tgttgaacaa gtctggaaag 3840

aaatgcataa acttttgcca ttctcaccgg attcagtcgt cactcatggt gatttctcac 3900

ttgataacct tatttttgac gaggggaaat taataggttg tattgatgtt ggacgagtcg 3960

gaatcgcaga ccgataccag gatcttgcca tcctatggaa ctgcctcggt gagttttctc 4020

cttcattaca gaaacggctt tttcaaaaat atggtattga taatcctgat atgaataaat 4080

tgcagtttca tttgatgctc gatgagtttt tctaatcaga attggttaat tggttgtaac 4140

actggcagag cattacgctg acttgacggg acggcggctt tgttgaataa atcgaacttt 4200

tgctgagttg aaggatcaga tcacgcatct tcccgacaac gcagaccgtt ccgtggcaaa 4260

gcaaaagttc aaaatcacca actggtccac ctacaacaaa gctctcatca accgtggcgg 4320

ggatcc 4326

<210> 8
<211> 5
<212> DNA
<213> Artificial

<220>
<223> An artificially synthesized nucleotide sequence

<400> 8
aacct 5

<210> 9
<211> 14
<212> RNA
<213> Artificial

<220>

<223>  An artificially synthesized nucleotide sequence

<400>  9
aucaccuccu uucu                                                          14


<210>  10
<211>  6
<212>  RNA
<213>  Artificial

<220>
<223>  An artificially synthesized nucleotide sequence

<400>  10
aggagg                                                                    6


<210>  11
<211>  19
<212>  DNA
<213>  Artificial

<220>
<223>  An artificially synthesized nucleotide sequence

<400>  11
ctagcaagga ggccaacct                                                     19


<210>  12
<211>  22
<212>  DNA
<213>  Artificial

<220>
<223>  An artificially synthesized nucleotide sequence

<400>  12
ctagcaagga ggccaacata tg                                                 22


<210>  13
<211>  36
<212>  DNA
<213>  Artificial

<220>
<223>  An artificially synthesized nucleotide sequence

<400>  13
gtgcatatga gcgagtacac ccagcaatac cgcgtc                                  36


<210>  14
<211>  37
<212>  DNA
<213>  Artificial

<220>

<223>  An artificially synthesized nucleotide sequence

<400>  14
tcctctagat cagagctgaa cggcctcctc cgaacgc                                37


<210>  15
<211>  6
<212>  DNA
<213>  Artificial

<220>
<223>  An artificially synthesized nucleotide sequence

<400>  15
ttgacc                                                                   6


<210>  16
<211>  6
<212>  DNA
<213>  Artificial

<220>
<223>  An artificially synthesized nucleotide sequence

<400>  16
gacagt                                                                   6


<210>  17
<211>  28
<212>  DNA
<213>  Artificial

<220>
<223>  An artificially synthesized nucleotide sequence

<400>  17
ggcgtcgacc gagggtaggg cgggagtg                                          28


<210>  18
<211>  29
<212>  DNA
<213>  Artificial

<220>
<223>  An artificially synthesized nucleotide sequence

<400>  18
tcgactagtg gcgtgatggt tcaactgtc                                         29


<210>  19
<211>  29
<212>  DNA
<213>  Artificial

<220>

<223>  An artificially synthesized nucleotide sequence

<400>  19
agccatatgg aaacatcctt tcgtttgcg                                          29


<210>  20
<211>  5445
<212>  DNA
<213>  Artificial

<220>
<223>  An artificially synthesized vector sequence

<400>  20
tctagcaagg aggccaacat atgagcgagt acacccagca ataccgcgtc gccgtcgtgc      60

aggccgagcc ggtctggaac gacgtggcag gcggcatcga gaagaccatc cggctggcca     120

ggcaggcagc agagggcggc gccgacctca tcgcgttccc cgaggtgtgg atccccggct     180

acccgtggtt cctctggctg gactccgtgg cgtggcagtc ccggttcgtg ctgccgtaca     240

tggagaactc gatcgagctc ggcagcgcgg agcaccgcga gctggagcgc gcagcggcgg     300

aggtcgggat cgcgatgagc ctcggcttca gcgagcggga tcgaggttcc ctgtacatcg     360

cgcaggcgtt catcgacagc gacggcgtga ccaggaccac ccggcgcaag ctgaagccga     420

cgcacgtgga gcgcacgctg ttcggggagg gcgacggcgc cgatatccag gtgatcgaca     480

ccgacttcgg ccggctcggc cagctcaact gctgggagca cctgcagccg ctggcgaagt     540

acgccatgtt ctcgcagggt gagcaggtcc acgtggccgc gtggccgagc ttctccatct     600

tcgaggatgc cgtctacgcc ctcggtccag aggtgaacgt gggggcggca cggcagtacg     660

cagtggaggg ccagtgcttc gtgctctcac cgtgcgggat ggtggggcag gcgggacagg     720

aactcctggc cgacaccgag ctgaagaagg acctgctgcg gctcggcggt gggcatgcgc     780

gcatcttcgg ccccgacggc cgccccctgg ccgagcccct cgcccccgat caggaggggc     840

tgctcttcgc cgacctggac catcgcgcca tcctggccgc caagaacgca gcggaccccg     900

tggggcacta ctcgcgcccc gacgtgttcc ggctgcactt cgacaacacg gccaagccca     960

tggtggtgga gggtgccatg gcggccggcg gtgccgacga gagggatgaa gccgacggca    1020

tcgcagccga gggcatcgtg cccacccgca gctctgcgcc cggtgatggc gatggcgtcg    1080

gggcggacga cggcgctgcg tcgcacgccg gcaggcgttc ggaggaggcc gttcagctct    1140

gatctagagc ggtctgcggg ccacctacaa cggggtggcc cgcagaccgc gtgtctggcg    1200

acacgccctg gacatgcgga agggcaccgc gccaacggtg ccctatcctg aagttgaact    1260

gaaacagtga gtccattcta gcgcctgcgc caacgggtgc caatggagcg cccagcgatg    1320

gaagacctgg gtggggcctg agacgcggcc ccgtatcaac atcaccagcc gccttacacg    1380

cggtgctccc cgggtcagag ccggttcctt ttgccgagcg cacagacggg ggccgtcacc 1440

agggatcgca agacctggat agcgaaggag tgccaagccc tacagtgccg ctcgtggaca 1500

gcagccctgc cacgcagcga gcaccggagc tgtgcgcaca tgccgccact gcccacgagt 1560

aatgccgtga gccttcttca accacccatg aggtggtcaa ccgatgcgac cgatcgaccg 1620

accgggctga tccgcaccgt gttccaagat gccttagacg ttccccaccc cacccttctt 1680

caccgaaggt gtgccccggg aacgtttaag gcatccccct gccctccgct ccctccctcc 1740

ctcccaggct ggccggttgg tccactcctg aacagcacca cagaacggac tggggaagag 1800

cggagggcag gaaaagaatg agcgcgagcg aagcgagctc ctttccccgc cacagctaga 1860

acaccaggcc ctttgggctg gtcggtgacc gaggaagaac ctcgttcacg catcccacct 1920

tgggtaggtg ggggatcgtc ttccgctgcg ctgcagacga tgcgtggtgg tgccctggcc 1980

ctggctctgt cctcctgcgt cggtcaggcc cagcacacca ccacgccccc tgttccagcg 2040

cgctgttctc ctccttcgtc ggggcgctga aacggggatg caaggtatga ttgtccggaa 2100

cggaaaacct ggcgcaccgc tcgttcctcg ctgccaggtg ttcccgttcc ggtcaatccc 2160

cttgctcctg cggagggctg gtctcgaccc cttggcgggg tctcgaaagg gtggtcccgt 2220

tgggcttgtt ctcacgtcgt tcgacctcgc cgggcacggt cccgtcgggg tccgggaacc 2280

ggtgggaggg gctgctcctc gagcagcccc tcgcctcacc ggacccggaa ccaaccgata 2340

cagcgtcggc gcctcggatg cggggtgctg gtgggcggcc ggtggcggtg gagccgcgga 2400

cccggcgggt gtcgatgtcg ttgaccgagg ctgagcaccg ggcgtgggcg gaggccgccg 2460

gcgggcaacg gttgtcggtg tgggctcggg aacgagtcca ggaacagctt gatgcggaga 2520

gtccgcggat gggtgaggtg caggagttgg cgaggatccg ggcggatctg gcgcgggtgg 2580

ggtcgaatct gaaccagttg atgcgtgcgg tgaaccaggg gcaggtcgtc ccgaccccgc 2640

agctgtatga ggccgtggac gcgctggcga gtcagctggg agtagtgcgg aggcagttgc 2700

cgtgatcgcg aagatcaccc ggggggcgaa cccgggcaac atcggggctt atctgcatgg 2760

gccggggcat gccaatgagc acttctacga acgcaacggg ggccggtatg ccggtggtgt 2820

ggtcgtgggc gggaacgtgt tcgtgaccgg ggatgcggat gagaaggcgt gggtgaagga 2880

gatgcgcgcg gcgatgcgca cccgcccgga gatcacgaaa ccgatctggc aggcctcttt 2940

gcgcaacacg gctggggatc ggcggttgac ggatgcggag tgggcgcagg caggccagac 3000

gtttgcggag tcgatggggt tcgcggagca cccgtgggtg atggtccgcc acgccgatga 3060

ccacgtgcat gtggtggtgt gccgggtcag tgacgagggg acggtgtggc acggtcgcaa 3120

cgaccgccgc gccgcccaaa gcgcgtgcac ccgtctggag ttggagcacg gtttgcagac 3180

cgcaccgcgg cgccgtgagg cgggacgcgg caaacgttca gtgctcgcgg tgcgtcagca 3240

gcaggagcag caggctcagg tggtggcggc ccgccgcgcg gagtgggagc agatccgcaa 3300

gttcgtggag ctggacaacc cggcaccccg gaaacagtcc ccggcacccc aacagcagca 3360

gtgggtagcc ccacctcatt cggtggagca ggtgcagcac cttcgtcgtc ccggccccgg 3420

ccggggggat cgtgggcggg gcctgtagct gggcttagag gtcgcgcggg tagggcgcgg 3480

cttgtcgggg atgtgacgca gctgtctctt atacacatct caaccatcat cgatgaattg 3540

cttcgttaat acagatgtag gtgttccaca gggtagccag cagcatcctg cgatgcagat 3600

ccggatgcca tttcattacc tctttctccg cacccgacat agatccgaag atcagcagtt 3660

caacctgttg atagtacgta ctaagctctc atgtttcacg tactaagctc tcatgtttaa 3720

cgtactaagc tctcatgttt aacgaactaa accctcatgg ctaacgtact aagctctcat 3780

ggctaacgta ctaagctctc atgtttcacg tactaagctc tcatgtttga acaataaaat 3840

taatataaat cagcaactta aatagcctct aaggttttaa gttttataag aaaaaaaaga 3900

atatataagg cttttaaagc ttttaaggtt taacggttgt ggacaacaag ccagggatct 3960

gccatttcat tacctctttc tccgcacccg acatagatcc ggaacataat ggtgcagggc 4020

gctgacttcc gcgtttccag actttacgaa acacggaaac cgaagaccat tcatgttgtt 4080

gctcaggtcg cagacgtttt gcagcagcag tcgcttcacg ttcgctcgcg tatcggtgat 4140

tcattctgct aaccagtaag gcaaccccgc cagcctagcc gggtcctcaa cgacaggagc 4200

acgatcatgc gcacccgtgg ccaggaccca acgctgcccg agatgcgccg cgtgcggctg 4260

ctggagatgg cggacgcgat ggatatgttc tgccaagggt tggtttgcgc attcacaggg 4320

tgtctcaaaa tctctgatgt tacattgcac aagataaaaa tatatcatca tgaacaataa 4380

aactgtctgc ttacataaac agtaatacaa ggggtgttat gagccatatt caacgggaaa 4440

cgtcttgctc gaggccgcga ttaaattcca acatggatgc tgatttatat gggtataaat 4500

gggctcgcga taatgtcggg caatcaggtg cgacaatcta tcgattgtat gggaagcccg 4560

atgcgccaga gttgtttctg aaacatggca aaggtagcgt tgccaatgat gttacagatg 4620

agatggtcag actaaactgg ctgacggaat ttatgcctct tccgaccatc aagcatttta 4680

tccgtactcc tgatgatgca tggttactca ccactgcgat ccccggaaaa acagcattcc 4740

aggtattaga agaatatcct gattcaggtg aaaatattgt tgatgcgctg gcagtgttcc 4800

tgcgccggtt gcattcgatt cctgtttgta attgtccttt taacagcgat cgcgtatttc 4860

gtctcgctca ggcgcaatca cgaatgaata acggtttggt tgatgcgagt gattttgatg 4920

acgagcgtaa tggctggcct gttgaacaag tctggaaaga aatgcataaa cttttgccat 4980

tctcaccgga ttcagtcgtc actcatggtg atttctcact tgataacctt atttttgacg 5040

aggggaaatt aataggttgt attgatgttg gacgagtcgg aatcgcagac cgataccagg 5100

atcttgccat cctatggaac tgcctcggtg agttttctcc ttcattacag aaacggcttt 5160

ttcaaaaata tggtattgat aatcctgata tgaataaatt gcagtttcat ttgatgctcg 5220

atgagttttt ctaatcagaa ttggttaatt ggttgtaaca ctggcagagc attacgctga 5280

cttgacggga cggcggcttt gttgaataaa tcgaactttt gctgagttga aggatcagat 5340

cacgcatctt cccgacaacg cagaccgttc cgtggcaaag caaaagttca aaatcaccaa 5400

ctggtccacc tacaacaaag ctctcatcaa ccgtggcggg gatcc 5445

<210> 21
<211> 4681
<212> DNA
<213> Artificial

<220>
<223> An artificially synthesized vector sequence

<400> 21
tcatgaacaa taaaactgtc tgcttacata aacagtaata caaggggtgt tatgagccat 60

attcaacggg aaacgtcttg ctcgaggccg cgattaaatt ccaacatgga tgctgattta 120

tatgggtata aatgggctcg cgataatgtc gggcaatcag gtgcgacaat ctatcgattg 180

tatgggaagc ccgatgcgcc agagttgttt ctgaaacatg gcaaaggtag cgttgccaat 240

gatgttacag atgagatggt cagactaaac tggctgacgg aatttatgcc tcttccgacc 300

atcaagcatt ttatccgtac tcctgatgat gcatggttac tcaccactgc gatccccgga 360

aaaacagcat tccaggtatt agaagaatat cctgattcag gtgaaaatat tgttgatgcg 420

ctggcagtgt tcctgcgccg gttgcattcg attcctgttt gtaattgtcc ttttaacagc 480

gatcgcgtat ttcgtctcgc tcaggcgcaa tcacgaatga ataacggttt ggttgatgcg 540

agtgattttg atgacgagcg taatggctgg cctgttgaac aagtctggaa agaaatgcat 600

aaacttttgc cattctcacc ggattcagtc gtcactcatg gtgatttctc acttgataac 660

cttatttttg acgaggggaa attaataggt tgtattgatg ttggacgagt cggaatcgca 720

gaccgatacc aggatcttgc catcctatgg aactgcctcg gtgagttttc tccttcatta 780

cagaaacggc tttttcaaaa atatggtatt gataatcctg atatgaataa attgcagttt 840

catttgatgc tcgatgagtt tttctaatca gaattggtta attggttgta acactggcag 900

agcattacgc tgacttgacg ggacggcggc tttgttgaat aaatcgaact tttgctgagt 960

tgaaggatca gatcacgcat cttcccgaca acgcagaccg ttccgtggca aagcaaaagt 1020

tcaaaatcac caactggtcc acctacaaca aagctctcat caaccgtggc ggggatctcg 1080

aggtcgaccg agggtagggc gggagtgggc gcggggaacg tggtgtgacg gaacgtgacc 1140

ggggcccggg agtcgtacgc cagtggctga acatccttga cctgcggaac ggcggcgatg 1200

gacagttgaa ccatcacgcc actagttcta ggatcctcta gcaaggaggc caacatatga 1260

gcgagtacac ccagcaatac cgcgtcgccg tcgtgcaggc cgagccggtc tggaacgacg 1320

tggcaggcgg catcgagaag accatccggc tggccaggca ggcagcagag ggcggcgccg 1380

acctcatcgc gttccccgag gtgtggatcc ccggctaccc gtggttcctc tggctggact 1440

ccgtggcgtg gcagtcccgg ttcgtgctgc cgtacatgga gaactcgatc gagctcggca 1500

gcgcggagca ccgcgagctg gagcgcgcag cggcggaggt cgggatcgcg atgagcctcg 1560

gcttcagcga gcgggatcga ggttccctgt acatcgcgca ggcgttcatc gacagcgacg 1620

gcgtgaccag gaccacccgg cgcaagctga agccgacgca cgtggagcgc acgctgttcg 1680

gggagggcga cggcgccgat atccaggtga tcgacaccga cttcggccgg ctcggccagc 1740

tcaactgctg ggagcacctg cagccgctgg cgaagtacgc catgttctcg cagggtgagc 1800

aggtccacgt ggccgcgtgg ccgagcttct ccatcttcga ggatgccgtc tacgccctcg 1860

gtccagaggt gaacgtgggg gcggcacggc agtacgcagt ggagggccag tgcttcgtgc 1920

tctcaccgtg cgggatggtg gggcaggcgg gacaggaact cctggccgac accgagctga 1980

agaaggacct gctgcggctc ggcggtgggc atgcgcgcat cttcggcccc gacggccgcc 2040

ccctggccga gcccctcgcc cccgatcagg aggggctgct cttcgccgac ctggaccatc 2100

gcgccatcct ggccgccaag aacgcagcgg accccgtggg gcactactcg cgccccgacg 2160

tgttccggct gcacttcgac aacacggcca agcccatggt ggtggagggt gccatggcgg 2220

ccggcggtgc cgacgagagg gatgaagccg acggcatcgc agccgagggc atcgtgccca 2280

cccgcagctc tgcgcccggt gatggcgatg gcgtcggggc ggacgacggc gctgcgtcgc 2340

acgccggcag gcgttcggag gaggccgttc agctctgatc tagagcggtc tgcgggccac 2400

ctacaacggg gtggcccgca gaccgcgtgt ctggcgacac gccctggaca tgcggaaggg 2460

caccgcgcca acggtgccct atcctgaagt tgaactgaaa cagtgagtcc attctagcgc 2520

ctgcgccaac gggtgccaat ggagcgccca gcgatggaag acctgggtgg ggcctgagac 2580

gcggccccgt atcaacatca ccagccgcct tacacgcggt gctccccggg tcagagccgg 2640

ttccttttgc cgagcgcaca gacgggggcc gtcaccaggg atcgcaagac ctggatagcg 2700

aaggagtgcc aagccctaca gtgccgctcg tggacagcag ccctgccacg cagcgagcac 2760

cggagctgtg cgcacatgcc gccactgccc acgagtaatg ccgtgagcct tcttcaacca 2820

cccatgaggt ggtcaaccga tgcgaccgat cgaccgaccg ggctgatccg caccgtgttc 2880

caagatgcct tagacgttcc ccaccccacc cttcttcacc gaaggtgtgc cccgggaacg 2940

tttaaggcat ccccctgccc tccgctccct ccctccctcc caggctggcc ggttggtcca 3000

ctcctgaaca gcaccacaga acggactggg gaagagcgga gggcaggaaa agaatgagcg 3060

cgagcgaagc gagctccttt ccccgccaca gctagaacac caggcccttt gggctggtcg 3120

gtgaccgagg aagaacctcg ttcacgcatc ccaccttggg taggtggggg atcgtcttcc 3180

gctgcgccct ctttgcgcaa cacggctggg gatcggcggt tgacggatgc ggagtgggcg 3240

caggcaggcc agacgtttgc ggagtcgatg gggttcgcgg agcacccgtg ggtgatggtc 3300

cgccacgccg atgaccacgt gcatgtggtg gtgtgccggg tcagtgacga ggggacggtg 3360

tggcacggtc gcaacgaccg ccgcgccgcc caaagcgcgt gcacccgtct ggagttggag 3420

cacggtttgc agaccgcacc gcggcgccgt gaggcgggac gcggcaaacg ttcagtgctc 3480

gcggtgcgtc agcagcagga gcagcaggct caggtggtgg cggcccgccg cgcggagtgg 3540

gagcagatcc gcaagttcgt ggagctggac aacccggcac cccggaaaca gtccccggca 3600

ccccaacagc agcagtgggt agccccacct cattcggtgg agcaggtgca gcaccttcgt 3660

cgtcccggcc ccggccgggg ggatcgtggg cggggcctgt agctgggctt agaggtcgcg 3720

cgggtagggc gcggcttgtc ggggatgtga cgcagctgtc tcttatacac atctcaacca 3780

tcatcgctgc attaatgaat cggccaacgc gcggggagag gcggtttgcg tattgggcgc 3840

tcttccgctt cctcgctcac tgactcgctg cgctcggtcg ttcggctgcg gcgagcggta 3900

tcagctcact caaaggcggt aatacggtta tccacagaat caggggataa cgcaggaaag 3960

aacatgtgag caaaaggcca gcaaaaggcc aggaaccgta aaaaggccgc gttgctggcg 4020

tttttccata ggctccgccc ccctgacgag catcacaaaa atcgacgctc aagtcagagg 4080

tggcgaaacc cgacaggact ataaagatac caggcgtttc cccctggaag ctccctcgtg 4140

cgctctcctg ttccgaccct gccgcttacc ggatacctgt ccgcctttct cccttcggga 4200

agcgtggcgc tttctcatag ctcacgctgt aggtatctca gttcggtgta ggtcgttcgc 4260

tccaagctgg gctgtgtgca cgaacccccc gttcagcccg accgctgcgc cttatccggt 4320

aactatcgtc ttgagtccaa cccggtaaga cacgacttat cgccactggc agcagccact 4380

ggtaacagga ttagcagagc gaggtatgta ggcggtgcta cagagttctt gaagtggtgg 4440

cctaactacg gctacactag aaggacagta tttggtatct gcgctctgct gaagccagtt 4500

accttcggaa aaagagttgg tagctcttga tccggcaaac aaaccaccgc tggtagcggt 4560

ggtttttttg tttgcaagca gcagattacg cgcagaaaaa aaggatctca agaagatcct 4620

ttgatctttt ctacggggtc tgacgctcag tggaacgaaa actcacgtta agggattttg 4680

g 4681

<210> 22
<211> 4668
<212> DNA
<213> Artificial

<220>
<223> An artificially synthesized vector sequence

<400> 22
tcatgaacaa taaaactgtc tgcttacata aacagtaata caaggggtgt tatgagccat 60

attcaacggg aaacgtcttg ctcgaggccg cgattaaatt ccaacatgga tgctgattta 120

tatgggtata aatgggctcg cgataatgtc gggcaatcag gtgcgacaat ctatcgattg 180

tatgggaagc ccgatgcgcc agagttgttt ctgaaacatg gcaaaggtag cgttgccaat 240

gatgttacag atgagatggt cagactaaac tggctgacgg aatttatgcc tcttccgacc 300

atcaagcatt ttatccgtac tcctgatgat gcatggttac tcaccactgc gatccccgga 360

aaaacagcat tccaggtatt agaagaatat cctgattcag gtgaaaatat tgttgatgcg 420

ctggcagtgt tcctgcgccg gttgcattcg attcctgttt gtaattgtcc ttttaacagc 480

gatcgcgtat ttcgtctcgc tcaggcgcaa tcacgaatga ataacggttt ggttgatgcg 540

agtgattttg atgacgagcg taatggctgg cctgttgaac aagtctggaa agaaatgcat 600

aaacttttgc cattctcacc ggattcagtc gtcactcatg gtgatttctc acttgataac 660

cttatttttg acgaggggaa attaataggt tgtattgatg ttggacgagt cggaatcgca 720

gaccgatacc aggatcttgc catcctatgg aactgcctcg gtgagttttc tccttcatta 780

cagaaacggc tttttcaaaa atatggtatt gataatcctg atatgaataa attgcagttt 840

catttgatgc tcgatgagtt tttctaatca gaattggtta attggttgta acactggcag 900

agcattacgc tgacttgacg ggacggcggc tttgttgaat aaatcgaact tttgctgagt 960

tgaaggatca gatcacgcat cttcccgaca acgcagaccg ttccgtggca aagcaaaagt 1020

tcaaaatcac caactggtcc acctacaaca aagctctcat caaccgtggc ggggatctcg 1080

aggtcgaccg agggtagggc gggagtgggc gcggggaacg tggtgtgacg gaacgtgacc 1140

ggggcccggg agtcgtacgc cagtggctga acatccttga cctgcggaac ggcggcgatg 1200

gacagttgaa ccatcacgcc cgcaaacgaa aggatgtttc catatgagcg agtacaccca 1260

gcaataccgc gtcgccgtcg tgcaggccga gccggtctgg aacgacgtgg caggcggcat 1320

cgagaagacc atccggctgg ccaggcaggc agcagagggc ggcgccgacc tcatcgcgtt 1380

ccccgaggtg tggatccccg gctacccgtg gttcctctgg ctggactccg tggcgtggca 1440

gtcccggttc gtgctgccgt acatggagaa ctcgatcgag ctcggcagcg cggagcaccg 1500

cgagctggag cgcgcagcgg cggaggtcgg gatcgcgatg agcctcggct tcagcgagcg 1560

ggatcgaggt tccctgtaca tcgcgcaggc gttcatcgac agcgacggcg tgaccaggac 1620

cacccggcgc aagctgaagc cgacgcacgt ggagcgcacg ctgttcgggg agggcgacgg 1680

cgccgatatc caggtgatcg acaccgactt cggccggctc ggccagctca actgctggga 1740

gcacctgcag ccgctggcga agtacgccat gttctcgcag ggtgagcagg tccacgtggc 1800

cgcgtggccg agcttctcca tcttcgagga tgccgtctac gccctcggtc cagaggtgaa 1860

cgtgggggcg gcacggcagt acgcagtgga gggccagtgc ttcgtgctct caccgtgcgg 1920

gatggtgggg caggcgggac aggaactcct ggccgacacc gagctgaaga aggacctgct 1980

gcggctcggc ggtgggcatg cgcgcatctt cggccccgac ggccgccccc tggccgagcc 2040

cctcgccccc gatcaggagg ggctgctctt cgccgacctg gaccatcgcg ccatcctggc 2100

cgccaagaac gcagcggacc ccgtggggca ctactcgcgc cccgacgtgt tccggctgca 2160

cttcgacaac acggccaagc ccatggtggt ggagggtgcc atggcggccg gcggtgccga 2220

cgagagggat gaagccgacg gcatcgcagc cgagggcatc gtgcccaccc gcagctctgc 2280

gcccggtgat ggcgatggcg tcggggcgga cgacggcgct gcgtcgcacg ccggcaggcg 2340

ttcggaggag gccgttcagc tctgatctag agcggtctgc gggccaccta caacggggtg 2400

gcccgcagac cgcgtgtctg gcgacacgcc ctggacatgc ggaagggcac cgcgccaacg 2460

gtgccctatc ctgaagttga actgaaacag tgagtccatt ctagcgcctg cgccaacggg 2520

tgccaatgga gcgcccagcg atggaagacc tgggtggggc ctgagacgcg gccccgtatc 2580

aacatcacca gccgccttac acgcggtgct ccccgggtca gagccggttc cttttgccga 2640

gcgcacagac gggggccgtc accagggatc gcaagacctg gatagcgaag gagtgccaag 2700

ccctacagtg ccgctcgtgg acagcagccc tgccacgcag cgagcaccgg agctgtgcgc 2760

acatgccgcc actgcccacg agtaatgccg tgagccttct tcaaccaccc atgaggtggt 2820

caaccgatgc gaccgatcga ccgaccgggc tgatccgcac cgtgttccaa gatgccttag 2880

acgttcccca ccccaccctt cttcaccgaa ggtgtgcccc gggaacgttt aaggcatccc 2940

cctgccctcc gctccctccc tccctcccag gctggccggt tggtccactc ctgaacagca 3000

ccacagaacg gactggggaa gagcggaggg caggaaaaga atgagcgcga gcgaagcgag 3060

ctcctttccc cgccacagct agaacaccag gccctttggg ctggtcggtg accgaggaag 3120

aacctcgttc acgcatccca ccttgggtag gtgggggatc gtcttccgct gcgccctctt 3180

tgcgcaacac ggctggggat cggcggttga cggatgcgga gtgggcgcag gcaggccaga 3240

cgtttgcgga gtcgatgggg ttcgcggagc acccgtgggt gatggtccgc cacgccgatg 3300

accacgtgca tgtggtggtg tgccgggtca gtgacgaggg gacggtgtgg cacggtcgca 3360

acgaccgccg cgccgcccaa agcgcgtgca cccgtctgga gttggagcac ggtttgcaga 3420

ccgcaccgcg gcgccgtgag gcgggacgcg gcaaacgttc agtgctcgcg gtgcgtcagc 3480

agcaggagca gcaggctcag gtggtggcgg cccgccgcgc ggagtgggag cagatccgca 3540

agttcgtgga gctggacaac ccggcacccc ggaaacagtc cccggcaccc caacagcagc 3600

agtgggtagc cccacctcat tcggtggagc aggtgcagca ccttcgtcgt cccggccccg 3660

gccgggggga tcgtgggcgg ggcctgtagc tgggcttaga ggtcgcgcgg gtagggcgcg 3720

gcttgtcggg gatgtgacgc agctgtctct tatacacatc tcaaccatca tcgctgcatt 3780

aatgaatcgg ccaacgcgcg gggagaggcg gtttgcgtat tgggcgctct tccgcttcct 3840

cgctcactga ctcgctgcgc tcggtcgttc ggctgcggcg agcggtatca gctcactcaa 3900

aggcggtaat acggttatcc acagaatcag gggataacgc aggaaagaac atgtgagcaa 3960

aaggccagca aaaggccagg aaccgtaaaa aggccgcgtt gctggcgttt ttccataggc 4020

tccgcccccc tgacgagcat cacaaaaatc gacgctcaag tcagaggtgg cgaaacccga 4080

caggactata aagataccag gcgtttcccc ctggaagctc cctcgtgcgc tctcctgttc 4140

cgaccctgcc gcttaccgga tacctgtccg cctttctccc ttcgggaagc gtggcgcttt 4200

ctcatagctc acgctgtagg tatctcagtt cggtgtaggt cgttcgctcc aagctgggct 4260

gtgtgcacga accccccgtt cagcccgacc gctgcgcctt atccggtaac tatcgtcttg 4320

agtccaaccc ggtaagacac gacttatcgc cactggcagc agccactggt aacaggatta 4380

gcagagcgag gtatgtaggc ggtgctacag agttcttgaa gtggtggcct aactacggct 4440

acactagaag gacagtattt ggtatctgcg ctctgctgaa gccagttacc ttcggaaaaa 4500

gagttggtag ctcttgatcc ggcaaacaaa ccaccgctgg tagcggtggt ttttttgttt 4560

gcaagcagca gattacgcgc agaaaaaaag gatctcaaga agatcctttg atcttttcta 4620

cggggtctga cgctcagtgg aacgaaaact cacgttaagg gattttgg 4668

<210> 23
<211> 3562
<212> DNA
<213> Artificial

<220>
<223> An artificially synthesized vector sequence

<400> 23
tcatgaacaa taaaactgtc tgcttacata aacagtaata caaggggtgt tatgagccat 60

attcaacggg aaacgtcttg ctcgaggccg cgattaaatt ccaacatgga tgctgattta 120

tatgggtata aatgggctcg cgataatgtc gggcaatcag gtgcgacaat ctatcgattg 180

tatgggaagc ccgatgcgcc agagttgttt ctgaaacatg gcaaaggtag cgttgccaat 240

gatgttacag atgagatggt cagactaaac tggctgacgg aatttatgcc tcttccgacc 300

atcaagcatt ttatccgtac tcctgatgat gcatggttac tcaccactgc gatccccgga 360

aaaacagcat tccaggtatt agaagaatat cctgattcag gtgaaaatat tgttgatgcg 420

ctggcagtgt tcctgcgccg gttgcattcg attcctgttt gtaattgtcc ttttaacagc 480

gatcgcgtat ttcgtctcgc tcaggcgcaa tcacgaatga ataacggttt ggttgatgcg 540

agtgattttg atgacgagcg taatggctgg cctgttgaac aagtctggaa agaaatgcat 600

aaacttttgc cattctcacc ggattcagtc gtcactcatg gtgatttctc acttgataac 660

cttatttttg acgaggggaa attaataggt tgtattgatg ttggacgagt cggaatcgca 720

gaccgatacc aggatcttgc catcctatgg aactgcctcg gtgagttttc tccttcatta 780

cagaaacggc tttttcaaaa atatggtatt gataatcctg atatgaataa attgcagttt 840

catttgatgc tcgatgagtt tttctaatca gaattggtta attggttgta acactggcag 900

agcattacgc tgacttgacg ggacggcggc tttgttgaat aaatcgaact tttgctgagt 960

tgaaggatca gatcacgcat cttcccgaca acgcagaccg ttccgtggca aagcaaaagt 1020

tcaaaatcac caactggtcc acctacaaca aagctctcat caaccgtggc ggggatctcg 1080

aggtcgaccg agggtagggc gggagtgggc gcggggaacg tggtgtgacg gaacgtgacc 1140

ggggcccggg agtcgtacgc cagtggctga acatccttga cctgcggaac ggcggcgatg 1200

gacagttgaa ccatcacgcc actagttcta ggatcctcta gcaaggaggc caacatatgt 1260

ctagagcggt ctgcgggcca cctacaacgg ggtggcccgc agaccgcgtg tctggcgaca 1320

cgccctggac atgcggaagg gcaccgcgcc aacggtgccc tatcctgaag ttgaactgaa 1380

acagtgagtc cattctagcg cctgcgccaa cgggtgccaa tggagcgccc agcgatggaa 1440

gacctgggtg gggcctgaga cgcggccccg tatcaacatc accagccgcc ttacacgcgg 1500

tgctccccgg gtcagagccg gttccttttg ccgagcgcac agacgggggc cgtcaccagg 1560

gatcgcaaga cctggatagc gaaggagtgc caagccctac agtgccgctc gtggacagca 1620

gccctgccac gcagcgagca ccggagctgt gcgcacatgc cgccactgcc cacgagtaat 1680

gccgtgagcc ttcttcaacc acccatgagg tggtcaaccg atgcgaccga tcgaccgacc 1740

gggctgatcc gcaccgtgtt ccaagatgcc ttagacgttc cccaccccac ccttcttcac 1800

cgaaggtgtg ccccgggaac gtttaaggca tccccctgcc ctccgctccc tccctccctc 1860

ccaggctggc cggttggtcc actcctgaac agcaccacag aacggactgg ggaagagcgg 1920

agggcaggaa aagaatgagc gcgagcgaag cgagctcctt tccccgccac agctagaaca 1980

ccaggccctt tgggctggtc ggtgaccgag gaagaacctc gttcacgcat cccaccttgg 2040

gtaggtgggg gatcgtcttc cgctgcgccc tctttgcgca acacggctgg ggatcggcgg 2100

ttgacggatg cggagtgggc gcaggcaggc cagacgtttg cggagtcgat ggggttcgcg 2160

gagcacccgt gggtgatggt ccgccacgcc gatgaccacg tgcatgtggt ggtgtgccgg 2220

gtcagtgacg aggggacggt gtggcacggt cgcaacgacc gccgcgccgc ccaaagcgcg 2280

tgcacccgtc tggagttgga gcacggtttg cagaccgcac cgcggcgccg tgaggcggga 2340

cgcggcaaac gttcagtgct cgcggtgcgt cagcagcagg agcagcaggc tcaggtggtg 2400

gcggcccgcc gcgcggagtg ggagcagatc cgcaagttcg tggagctgga caacccggca 2460

ccccggaaac agtccccggc accccaacag cagcagtggg tagccccacc tcattcggtg 2520

gagcaggtgc agcaccttcg tcgtcccggc cccggccggg ggatcgtggg gcggggcctg 2580

tagctgggct tagaggtcgc gcgggtaggg cgcggcttgt cggggatgtg acgcagctgt 2640

ctcttataca catctcaacc atcatcgctg cattaatgaa tcggccaacg cgcggggaga 2700

ggcggtttgc gtattgggcg ctcttccgct tcctcgctca ctgactcgct gcgctcggtc 2760

gttcggctgc ggcgagcggt atcagctcac tcaaaggcgg taatacggtt atccacagaa 2820

tcaggggata acgcaggaaa gaacatgtga gcaaaaggcc agcaaaaggc caggaaccgt 2880

aaaaaggccg cgttgctggc gtttttccat aggctccgcc cccctgacga gcatcacaaa 2940

aatcgacgct caagtcagag gtggcgaaac ccgacaggac tataaagata ccaggcgttt 3000

ccccctggaa gctccctcgt gcgctctcct gttccgaccc tgccgcttac cggatacctg 3060

tccgcctttc tcccttcggg aagcgtggcg ctttctcata gctcacgctg taggtatctc 3120

agttcggtgt aggtcgttcg ctccaagctg ggctgtgtgc acgaaccccc cgttcagccc 3180

gaccgctgcg ccttatccgg taactatcgt cttgagtcca acccggtaag acacgactta 3240

tcgccactgg cagcagccac tggtaacagg attagcagag cgaggtatgt aggcggtgct 3300

acagagttct tgaagtggtg gcctaactac ggctacacta gaaggacagt atttggtatc 3360

tgcgctctgc tgaagccagt taccttcgga aaaagagttg gtagctcttg atccggcaaa 3420

caaaccaccg ctggtagcgg tggttttttt gtttgcaagc agcagattac gcgcagaaaa 3480

aaaggatctc aagaagatcc tttgatcttt tctacggggt ctgacgctca gtggaacgaa 3540

aactcacgtt aagggatttt gg 3562

<210> 24
<211> 4594
<212> DNA
<213> Artificial

<220>
<223> An artificially synthesized vector sequence

<400> 24
tcatgaacaa taaaactgtc tgcttacata aacagtaata caaggggtgt tatgagccat 60

attcaacggg aaacgtcttg ctcgaggccg cgattaaatt ccaacatgga tgctgattta 120

tatgggtata aatgggctcg cgataatgtc gggcaatcag gtgcgacaat ctatcgattg 180

tatgggaagc ccgatgcgcc agagttgttt ctgaaacatg gcaaaggtag cgttgccaat 240

gatgttacag atgagatggt cagactaaac tggctgacgg aatttatgcc tcttccgacc 300

atcaagcatt ttatccgtac tcctgatgat gcatggttac tcaccactgc gatccccgga 360

aaaacagcat tccaggtatt agaagaatat cctgattcag gtgaaaatat tgttgatgcg 420

ctggcagtgt tcctgcgccg gttgcattcg attcctgttt gtaattgtcc ttttaacagc 480

gatcgcgtat ttcgtctcgc tcaggcgcaa tcacgaatga ataacggttt ggttgatgcg 540

agtgattttg atgacgagcg taatggctgg cctgttgaac aagtctggaa agaaatgcat 600

aaacttttgc cattctcacc ggattcagtc gtcactcatg gtgatttctc acttgataac 660

cttatttttg acgaggggaa attaataggt tgtattgatg ttggacgagt cggaatcgca 720

gaccgatacc aggatcttgc catcctatgg aactgcctcg gtgagttttc tccttcatta 780

cagaaacggc tttttcaaaa atatggtatt gataatcctg atatgaataa attgcagttt 840

catttgatgc tcgatgagtt tttctaatca gaattggtta attggttgta acactggcag 900

agcattacgc tgacttgacg ggacggcggc tttgttgaat aaatcgaact tttgctgagt 960

tgaaggatca gatcacgcat cttcccgaca acgcagaccg ttccgtggca aagcaaaagt 1020

tcaaaatcac caactggtcc acctacaaca aagctctcat caaccgtggc ggggatctcg 1080

aggtcgacgg tatcgataag cttgatatcg aattcctgca gcccggggga tccactagtt 1140

ctaggatcct ctagcaagga ggccaacata tgagcgagta cacccagcaa taccgcgtcg 1200

ccgtcgtgca ggccgagccg gtctggaacg acgtggcagg cggcatcgag aagaccatcc 1260

ggctggccag gcaggcagca gagggcggcg ccgacctcat cgcgttcccc gaggtgtgga 1320

tccccggcta cccgtggttc ctctggctgg actccgtggc gtggcagtcc cggttcgtgc 1380

tgccgtacat ggagaactcg atcgagctcg gcagcgcgga gcaccgcgag ctggagcgcg 1440

cagcggcgga ggtcgggatc gcgatgagcc tcggcttcag cgagcgggat cgaggttccc 1500

tgtacatcgc gcaggcgttc atcgacagcg acggcgtgac caggaccacc cggcgcaagc 1560
tgaagccgac gcacgtggag cgcacgctgt tcggggaggg cgacggcgcc gatatccagg 1620
tgatcgacac cgacttcggc cggctcggcc agctcaactg ctgggagcac ctgcagccgc 1680
tggcgaagta cgccatgttc tcgcagggtg agcaggtcca cgtggccgcg tggccgagct 1740
tctccatctt cgaggatgcc gtctacgccc tcggtccaga ggtgaacgtg ggggcggcac 1800
ggcagtacgc agtggagggc cagtgcttcg tgctctcacc gtgcgggatg gtggggcagg 1860
cgggacagga actcctggcc gacaccgagc tgaagaagga cctgctgcgg ctcggcggtg 1920
ggcatgcgcg catcttcggc cccgacggcc gccccctggc cgagcccctc gcccccgatc 1980
aggaggggct gctcttcgcc gacctggacc atcgcgccat cctggccgcc aagaacgcag 2040
cggaccccgt ggggcactac tcgcgccccg acgtgttccg gctgcacttc gacaacacgg 2100
ccaagcccat ggtggtggag ggtgccatgg cggccggcgg tgccgacgag agggatgaag 2160
ccgacggcat cgcagccgag ggcatcgtgc ccacccgcag ctctgcgccc ggtgatggcg 2220
atggcgtcgg ggcggacgac ggcgctgcgt cgcacgccgg caggcgttcg gaggaggccg 2280
ttcagctctg atctagagcg gtctgcgggc cacctacaac ggggtggccc gcagaccgcg 2340
tgtctggcga cacgccctgg acatgcggaa gggcaccgcg ccaacggtgc cctatcctga 2400
agttgaactg aaacagtgag tccattctag cgcctgcgcc aacgggtgcc aatggagcgc 2460
ccagcgatgg aagacctggg tggggcctga gacgcggccc cgtatcaaca tcaccagccg 2520
ccttacacgc ggtgctcccc gggtcagagc cggttccttt tgccgagcgc acagacgggg 2580
gccgtcacca gggatcgcaa gacctggata gcgaaggagt gccaagccct acagtgccgc 2640
tcgtggacag cagccctgcc acgcagcgag caccggagct gtgcgcacat gccgccactg 2700
cccacgagta atgccgtgag ccttcttcaa ccacccatga ggtggtcaac cgatgcgacc 2760
gatcgaccga ccgggctgat ccgcaccgtg ttccaagatg ccttagacgt tccccacccc 2820
acccttcttc accgaaggtg tgccccggga acgtttaagg catccccctg ccctccgctc 2880
cctccctccc tcccaggctg gccggttggt ccactcctga acagcaccac agaacggact 2940
ggggaagagc ggagggcagg aaaagaatga gcgcgagcga agcgagctcc tttccccgcc 3000
acagctagaa caccaggccc tttgggctgg tcggtgaccg aggaagaacc tcgttcacgc 3060
atcccacctt gggtaggtgg gggatcgtct tccgctgcgc cctctttgcg caacacggct 3120
ggggatcggc ggttgacgga tgcggagtgg gcgcaggcag gccagacgtt tgcggagtcg 3180
atggggttcg cggagcaccc gtgggtgatg gtccgccacg ccgatgacca cgtgcatgtg 3240
gtggtgtgcc gggtcagtga cgaggggacg gtgtggcacg gtcgcaacga ccgccgcgcc 3300

gcccaaagcg cgtgcacccg tctggagttg gagcacggtt tgcagaccgc accgcggcgc 3360

cgtgaggcgg gacgcggcaa acgttcagtg ctcgcggtgc gtcagcagca ggagcagcag 3420

gctcaggtgg tggcggcccg ccgcgcggag tgggagcaga tccgcaagtt cgtggagctg 3480

gacaacccgg caccccggaa acagtccccg gcaccccaac agcagcagtg ggtagcccca 3540

cctcattcgg tggagcaggt gcagcacctt cgtcgtcccg gccccggccg gggggatcgt 3600

gggcggggcc tgtagctggg cttagaggtc gcgcgggtag ggcgcggctt gtcggggatg 3660

tgacgcagct gtctcttata cacatctcaa ccatcatcgc tgcattaatg aatcggccaa 3720

cgcgcgggga gaggcggttt gcgtattggg cgctcttccg cttcctcgct cactgactcg 3780

ctgcgctcgg tcgttcggct gcggcgagcg gtatcagctc actcaaaggc ggtaatacgg 3840

ttatccacag aatcagggga taacgcagga aagaacatgt gagcaaaagg ccagcaaaag 3900

gccaggaacc gtaaaaaggc cgcgttgctg gcgtttttcc ataggctccg cccccctgac 3960

gagcatcaca aaaatcgacg ctcaagtcag aggtggcgaa acccgacagg actataaaga 4020

taccaggcgt ttccccctgg aagctccctc gtgcgctctc ctgttccgac cctgccgctt 4080

accggatacc tgtccgcctt tctcccttcg ggaagcgtgg cgctttctca tagctcacgc 4140

tgtaggtatc tcagttcggt gtaggtcgtt cgctccaagc tgggctgtgt gcacgaaccc 4200

cccgttcagc ccgaccgctg cgccttatcc ggtaactatc gtcttgagtc caacccggta 4260

agacacgact tatcgccact ggcagcagcc actggtaaca ggattagcag agcgaggtat 4320

gtaggcggtg ctacagagtt cttgaagtgg tggcctaact acggctacac tagaaggaca 4380

gtatttggta tctgcgctct gctgaagcca gttaccttcg gaaaaagagt tggtagctct 4440

tgatccggca aacaaaccac cgctggtagc ggtggttttt ttgtttgcaa gcagcagatt 4500

acgcgcagaa aaaaaggatc tcaagaagat cctttgatct tttctacggg gtctgacgct 4560

cagtggaacg aaaactcacg ttaagggatt ttgg 4594

<210> 25
<211> 3404
<212> DNA
<213> Artificial

<220>
<223> An artificially synthesized vector sequence

<400> 25
tcatgaacaa taaaactgtc tgcttacata aacagtaata caaggggtgt tatgagccat 60

attcaacggg aaacgtcttg ctcgaggccg cgattaaatt ccaacatgga tgctgattta 120

tatgggtata aatgggctcg cgataatgtc gggcaatcag gtgcgacaat ctatcgattg 180

tatgggaagc ccgatgcgcc agagttgttt ctgaaacatg gcaaaggtag cgttgccaat 240

gatgttacag atgagatggt cagactaaac tggctgacgg aatttatgcc tcttccgacc 300

atcaagcatt ttatccgtac tcctgatgat gcatggttac tcaccactgc gatccccgga 360

aaaacagcat tccaggtatt agaagaatat cctgattcag gtgaaaatat tgttgatgcg 420

ctggcagtgt tcctgcgccg gttgcattcg attcctgttt gtaattgtcc ttttaacagc 480

gatcgcgtat ttcgtctcgc tcaggcgcaa tcacgaatga ataacggttt ggttgatgcg 540

agtgattttg atgacgagcg taatggctgg cctgttgaac aagtctggaa agaaatgcat 600

aaacttttgc cattctcacc ggattcagtc gtcactcatg gtgatttctc acttgataac 660

cttatttttg acgaggggaa attaataggt tgtattgatg ttggacgagt cggaatcgca 720

gaccgatacc aggatcttgc catcctatgg aactgcctcg gtgagttttc tccttcatta 780

cagaaacggc tttttcaaaa atatggtatt gataatcctg atatgaataa attgcagttt 840

catttgatgc tcgatgagtt tttctaatca gaattggtta attggttgta acactggcag 900

agcattacgc tgacttgacg ggacggcggc tttgttgaat aaatcgaact tttgctgagt 960

tgaaggatca gatcacgcat cttcccgaca acgcagaccg ttccgtggca aagcaaaagt 1020

tcaaaatcac caactggtcc acctacaaca aagctctcat caaccgtggc ggggatcctc 1080

tagcaaggag gccaacatat gtctagagcg gtctgcgggc cacctacaac ggggtggccc 1140

gcagaccgcg tgtctggcga cacgccctgg acatgcggaa gggcaccgcg ccaacggtgc 1200

cctatcctga agttgaactg aaacagtgag tccattctag cgcctgcgcc aacgggtgcc 1260

aatggagcgc ccagcgatgg aagacctggg tggggcctga gacgcggccc cgtatcaaca 1320

tcaccagccg ccttacacgc ggtgctcccc gggtcagagc cggttccttt tgccgagcgc 1380

acagacgggg gccgtcacca gggatcgcaa gacctggata gcgaaggagt gccaagccct 1440

acagtgccgc tcgtggacag cagccctgcc acgcagcgag caccggagct gtgcgcacat 1500

gccgccactg cccacgagta atgccgtgag ccttcttcaa ccacccatga ggtggtcaac 1560

cgatgcgacc gatcgaccga ccgggctgat ccgcaccgtg ttccaagatg ccttagacgt 1620

tccccacccc acccttcttc accgaaggtg tgccccggga acgtttaagg catccccctg 1680

ccctccgctc cctccctccc tcccaggctg gccggttggt ccactcctga acagcaccac 1740

agaacggact ggggaagagc ggagggcagg aaaagaatga gcgcgagcga agcgagctcc 1800

tttccccgcc acagctagaa caccaggccc tttgggctgg tcggtgaccg aggaagaacc 1860

tcgttcacgc atcccacctt gggtaggtgg gggatcgtct tccgctgcgc cctctttgcg 1920

caacacggct ggggatcggc ggttgacgga tgcggagtgg gcgcaggcag gccagacgtt 1980

tgcggagtcg atggggttcg cggagcaccc gtgggtgatg gtccgccacg ccgatgacca 2040

cgtgcatgtg gtggtgtgcc gggtcagtga cgaggggacg gtgtggcacg gtcgcaacga 2100

ccgccgcgcc gcccaaagcg cgtgcacccg tctggagttg gagcacggtt tgcagaccgc 2160

accgcggcgc cgtgaggcgg gacgcggcaa acgttcagtg ctcgcggtgc gtcagcagca 2220

ggagcagcag gctcaggtgg tggcggcccg ccgcgcggag tgggagcaga tccgcaagtt 2280

cgtggagctg gacaacccgg caccccggaa acagtccccg gcaccccaac agcagcagtg 2340

ggtagcccca cctcattcgg tggagcaggt gcagcacctt cgtcgtcccg gccccggccg 2400

gggggatcgt gggcggggcc tgtagctggg cttagaggtc gcgcgggtag ggcgcggctt 2460

gtcggggatg tgacgcagct gtctcttata cacatctcaa ccatcatcgc tgcattaatg 2520

aatcggccaa cgcgcgggga gaggcggttt gcgtattggg cgctcttccg cttcctcgct 2580

cactgactcg ctgcgctcgg tcgttcggct gcggcgagcg gtatcagctc actcaaaggc 2640

ggtaatacgg ttatccacag aatcagggga taacgcagga aagaacatgt gagcaaaagg 2700

ccagcaaaag gccaggaacc gtaaaaaggc cgcgttgctg gcgtttttcc ataggctccg 2760

cccccctgac gagcatcaca aaaatcgacg ctcaagtcag aggtggcgaa acccgacagg 2820

actataaaga taccaggcgt ttccccctgg aagctccctc gtgcgctctc ctgttccgac 2880

cctgccgctt accggatacc tgtccgcctt tctcccttcg ggaagcgtgg cgctttctca 2940

tagctcacgc tgtaggtatc tcagttcggt gtaggtcgtt cgctccaagc tgggctgtgt 3000

gcacgaaccc cccgttcagc ccgaccgctg cgccttatcc ggtaactatc gtcttgagtc 3060

caacccggta agacacgact tatcgccact ggcagcagcc actggtaaca ggattagcag 3120

agcgaggtat gtaggcggtg ctacagagtt cttgaagtgg tggcctaact acggctacac 3180

tagaaggaca gtatttggta tctgcgctct gctgaagcca gttaccttcg gaaaaagagt 3240

tggtagctct tgatccggca aacaaaccac cgctggtagc ggtggttttt ttgtttgcaa 3300

gcagcagatt acgcgcagaa aaaaaggatc tcaagaagat cctttgatct tttctacggg 3360

gtctgacgct cagtggaacg aaaactcacg ttaagggatt ttgg 3404

## Description

### Technical Field

The present invention relates to shuttle vectors that are replicable and allow inducible expression of a foreign gene in microorganisms belonging to the genus *Kocuria,* and uses thereof.

### Background Art

Processes for producing useful substances such as industrial materials using microorganisms generally have the characteristic of low energy consumption and less waste generation. Therefore, with concerns about exhaustion of resources and effects of emissions such as CO₂ on the environment in recent years, development of techniques for producing useful substances by utilizing the functions of microorganisms (bioprocesses) is needed as a basic manufacturing technology platform in environment-conscious recycling-oriented industrial systems.

To construct fossil resource-independent or environmentally-friendly production processes in the chemical industry with the ultimate aim of carbon neutrality, it is necessary to construct a chemical production system that includes a bio-refinery from biomass. To accomplish this, several technical breakthroughs are necessary, and in particular, it is necessary to expand the range of use and application range of bioprocesses that use microbial cells which are suitable for natural resource recycling. Currently, bioprocesses are basically performed in an aqueous reaction site, and this significantly narrows down the application range of bioprocesses because producible targets (chemicals) become limited. While the aqueous reaction site is suitable for conversion and production of hydrophilic substances, it is not suitable for conversion, reaction, or production of hydrophobic substances which account for a large segment of chemicals.

Consequently, techniques are essential for designing a microbial factory or a biocatalyst by freely coordinating an optimal enzyme gene and host cell, which may produce chemicals of interest in an optimal reaction site. There are few examples of studies relating to the functions of microbial reactions in a non-aqueous reaction site containing organic solvents and examples of industrial production in such reaction site. A microorganism that can be practically used for microbial conversion reactions in organic solvents is the microorganism *Kocuria rhizophila* NBRC 103217, as searched from the standpoint of maintaining cellular structure in organic solvents, and its genetic information has been elucidated (Non-patent Document 1). However, many sections including the microorganism's mechanism for durability are unknown.

To construct a microbial factory or a biocatalyst that has more durability in a non-aqueous reaction site, it is necessary to elucidate the mechanism for durability and design host cells based on that mechanism in parallel.

On the other hand, with the focus on enzyme reactions, the most important factor to achieve a cost-competitive production process is a reaction that produces an expensive product of high value from inexpensive raw materials, and enzymes that catalyze such reactions are required. Furthermore, in the production step of various chemicals, practical enzymes that catalyze reactions useful in producing functional groups necessary for conversion into chemicals with higher value are also required. Reactions that produce expensive products from inexpensive raw materials include carbon-to-carbon binding reactions. For example, it is thought that reactions that polymerize through carbon-to-carbon binding by an aldolase using inexpensive low-molecular weight compounds such as an aldehyde as the starting material and reactions that polymerize through fixation of inexpensive carbon dioxide gas (carbon dioxide fixation reaction) are useful.

Furthermore, when various chemicals are produced, reactions useful for production of functional groups necessary to convert into those chemicals include, for example, hydroxylation reaction and carbon oxygenation reaction. In the case of these reactions, since chemical substances that do not dissolve in water such as hydrocarbons are substantively used as the starting material in most cases, it is necessary to carry out the reaction in a non-aqueous reaction site, and enzymes that can be used in a non-aqueous system become necessary. However, the relationship between organic solvent resistance and enzyme including three-dimensional structure is not clear, and the relationship needs to be elucidated by using organic solvent-resistant enzymes obtained so far (for example, nitrilase) as models and observing their three-dimensional structure and changes in organic solvent resistance by mutations.

Accordingly, for smooth progression of microbial reactions in a non-aqueous reaction site, development of vectors capable of expressing a desired foreign protein (organic solvent-resistant enzyme) in a microorganism that maintains the cellular structure even in a non-aqueous system has been desired.

### [Prior Art Documents]

### [Non-patent Document]

[Non-patent Document 1] J. Bacteriol. Vol. 190, p 4139-4146, 2008.

### Disclosure of the Invention

### [Problems to be Solved by the Invention]

The present invention was achieved in view of the above circumstances. An objective of the present invention is to provide shuttle vectors that are replicable and allow inducible expression of a foreign protein in microorganisms belonging to the genus *Kocuria.*

### [Means for Solving the Problems]

To solve the above-mentioned problems, the present inventors constructed a transformation system which uses as a host the *Kocuria rhizophila* NBRC 103217 strain which maintains its cellular structure in various organic solvents and show little protein leakage.

First, the entire nucleotide sequence (2.7 Mb) of the *Kocuria rhizophila* NBRC 103217 genome was determined, and 2,356 ORFs were estimated from the nucleotide sequence. Based on the ORF information, the upstream and downstream sequences of the SD sequence that are most suitable for translation were predicted.

Next, a transformation system of the *Kocuria rhizophila* NBRC 103217 strain was established using the EZ-TN5<KAN-2>Tnp transposon. It was found out that the efficiency of transformation is greatly improved by using the NBRC 10317 strain bacterial cells grown until OD600 = 0.7 in an LB medium containing 1.5% glycine as the competent cells, and after incubation at 35°C for five minutes, they are used for electroporation. Furthermore, when a transposon containing not only a kanamycin resistance gene which is a selection marker but also the GFP gene was constructed and the *Kocuria rhizophila* NBRC 103217 strain was transformed, a transformant carrying the GFP gene could be obtained even though the transformation efficiency was 3% of that when a transposon carries only the kanamycin resistance gene.

Next, since a rescue plasmid of the *Kocuria varians* NBRC 15358-derived plasmid (pKV15358-1) is replicable in the *Kocuria rhizophila* NBRC 103217 strain, to produce a plasmid vector that is replicable in the *Kocuria rhizophila* NBRC 103217 strain, the pKV15358-3 vector (4.3 kb) for the *Kocuria rhizophila* NBRC 103217 strain was constructed by miniaturizing pKV15358-1 and adding the above-mentioned optimal-for-translation upstream and downstream sequences of the SD sequence in the *Kocuria rhizophila* NBRC 103217 strain. When a nitrilase gene derived from the *Arthrobacter* sp. F73 strain was expressed in the *Kocuria rhizophila* NBRC 103217 strain, nitrilase activity was shown even though the expression level was about 1% of that in *Escherichia coli.* When the same transformant was used in an ethyl acetate-water biphasic reaction in which mandelic acid production is not observed in *E. coli,* even in a system containing 60% ethyl acetate, it showed a 60% activity of that in an aqueous-phase reaction.

Furthermore, to obtain a promoter for increasing the expression level of a foreign gene, superoxide dismutase was discovered as one of the proteins highly produced in the stationary phase of culture. It was revealed that expression of the nitrilase gene was increased 150 times when a promoter region that expresses the superoxide dismutase-encoding gene was identified, cloned, and introduced into a vector for the *Kocuria rhizophila* NBRC 103217 strain.

More specifically, by developing novel shuttle vectors, the present inventors successfully induced expression of a foreign protein in microorganisms of the genus *Kocuria* which can maintain their cellular structure in non-aqueous systems and show little protein leakage, and thereby completed the present invention.

More specifically, the present invention provides:
[1] a vector capable of expressing a foreign protein in a microorganism, which comprises a polynucleotide comprising the nucleotide sequence of SEQ ID NO: 1, or a polynucleotide that hybridizes under stringent conditions with a complementary strand of a polynucleotide comprising the nucleotide sequence of SEQ ID NO: 1;
[2] the vector of [1], wherein the vector additionally comprises a polynucleotide comprising the nucleotide sequence of SEQ ID NO: 2, or a polynucleotide that hybridizes under stringent conditions with a complementary strand of a polynucleotide comprising the nucleotide sequence of SEQ ID NO: 2;
[3] the vector of [1] or [2], wherein the vector additionally comprises a polynucleotide comprising the nucleotide sequence of SEQ ID NO: 3 or 4, or a polynucleotide that hybridizes under stringent conditions with a complementary strand of a polynucleotide comprising the nucleotide sequence of SEQ ID NO: 3 or 4;
[4] the vector of any one of [1] to [3], wherein the microorganism in which a foreign protein is expressed is a microorganism belonging to the genus *Kocuria,* the genus *Micrococcus,* or the genus *Arthrobacter*;
[5] the vector of [4], wherein the microorganism belonging to the genus *Kocuria* is the *Kocuria rhizophila* NBRC 103217 strain;
[6] a recombinant vector in which a DNA encoding a foreign protein is inserted into the vector of any one of [1] to [5];
[7] a transformant in which a host is transformed with the vector of any one of [1] to [5] and the recombinant vector of [6];
[8] a method for producing a foreign protein, which comprises the steps of culturing the transformant of [7] and isolating a foreign protein from the obtained culture; and
[9] a method for producing a substance of interest, wherein the transformant of [7] is used as a biocatalyst for producing the substance of interest in a non-aqueous reaction site.

### Brief Description of the Drawings

Fig. 1 shows the category scores.
Fig. 2 shows the results of analyzing the distribution of all ORFs of the NBRC 103217 strain.
Fig. 3 depicts a two-dimensional electrophoresis.
Fig. 4 shows the results of MALDI-TOF MS.

### Mode for Carrying Out the Invention

The present invention provides shuttle vectors that are replicable and allow inducible expression of a foreign protein in microorganisms belonging to the genus *Kocuria.*

Vectors of the present invention carry a DNA region that is replicable in microorganisms of the genus *Kocuria,* and a drug marker that confers drug resistance to microorganisms of the genus *Kocuria.* Vectors of the present invention are shuttle vector plasmids that can be replicated in microorganisms of the genus *Kocuria,* and are efficient vectors for expressing a foreign protein in microorganisms of the genus *Kocuria.*

Shuttle vectors of the present invention include circular plasmids that can be obtained from the *Kocuria varians* NBRC15358 strain. By elucidating the DNA region involved in replication of these plasmids and producing plasmids containing that DNA region, such plasmids are replicable in microorganisms of the genus *Kocuria.*

The replicable DNA region in microorganisms belonging to the genus *Kocuria* is not particularly limited as long as it is a region that enables a vector to be replicated when a vector containing the region is introduced into a microorganism of the genus *Kocuria,* and includes, for example, an autonomously replicating region derived from an endogenous plasmid vector pKV15358-1 (SEQ ID NO: 5) carried by the *Kocuria varians* strain. The autonomously replicating region derived from pKV15358-1 may be the full-length pKV15358-1, but to simplify the genetic manipulations it is preferably a portion of pKV15358-1. A portion of pKV15358-1 includes a region comprising the nucleotide sequence of SEQ ID NO: 1. Furthermore, as long as it can function in microorganisms of the genus *Kocuria,* it may be a DNA region comprising a sequence with one or several nucleotide substitutions, deletions, insertions, and/or such in the nucleotide sequence of SEQ ID NO: 1, or a DNA region that hybridizes under stringent conditions with a polynucleotide comprising the nucleotide sequence of SEQ ID NO: 1 or a probe that may be prepared from this sequence. Herein, "several" refers to, for example, two to 50, preferably two to ten, and more preferably two to five. Stringent conditions include washing conditions for conventional Southern hybridization, which are 60°C and a salt concentration corresponding to 1x SSC, and 0.1% SDS, or preferably 0.1x SSC, and 0.1% SDS. One or several nucleotide substitutions, deletions, and/or insertions can be carried out by techniques known to those skilled in the art at the time of filing the application.

The above-described region necessary for autonomous replication in the pKV15358-1 vector can be identified by deletion experiments which use expression of an antibiotic resistance gene in antibiotic-sensitive host cells as an indicator. That is, after introducing a drug resistance gene such as a kanamycin resistance gene or an ampicillin resistance gene into the pKV15358-1 vector, the vector is digested with a single restriction enzyme or a combination of restriction enzymes that do not digest this gene, recircularized by a DNA ligase, and then used to transform drug-sensitive bacterial cells. Drug-resistant bacterial strains that are selected from these cells using drug resistance as the indicator carry the derived vector containing the autonomously replicating region of the pKV15358-1 vector; and the autonomously replicating region of the pKV15358-1 vector can be identified by comparing the restriction enzyme map of the vector.

In the present invention, a drug marker that confers resistance to microorganisms of the genus *Kocuria* is not particularly limited as long as it is a gene that confers drug resistance when it is introduced into the bacteria, and includes, for example, a kanamycin resistance gene, an ampicillin resistance gene, a neomycin resistance gene, or a chloramphenicol resistance gene.

Plasmid vectors of the present invention are vectors comprising the above-mentioned autonomously replicating region and a drug resistance marker, and such vectors specifically include pKV15358-2 (SEQ ID NO: 6). pKV15358-2 is a vector composed of a fragment comprising an autonomously replicating region obtained by cleaving the above-mentioned pKV15358-1 with the *Xba*I restriction enzyme, as well as a kanamycin resistance gene and such. The vector of the present invention may be a modified form of pKV15358-2. Examples of the modification include mutations at sites not involved in the replication ability or drug resistance, and deletions or substitutions of a portion of the restriction enzyme sites.

A vector of the present invention may further comprise an SD sequence derived from a microorganism belonging to the genus *Kocuria.* The SD sequence is not particularly limited, but is preferably, for example, the SD sequence of SEQ ID NO: 2. Vectors further comprising an SD sequence include pKV15358-3 (SEQ ID NO: 7).

Furthermore, a promoter that may function in microorganisms of the genus *Kocuria* can be further incorporated. The promoter is not particularly limited, and examples include a promoter that expresses a gene encoding superoxide dismutase.

In the present invention, microorganisms belonging to the genus *Kocuria* are not particularly limited, but a preferred example includes *Kocuria rhizophila* NBRC 103217.

The present invention relates to transformants produced by transforming a host with a shuttle vector for inducible expression in microorganisms of the genus *Kocuria* into which a foreign protein-encoding DNA is inserted.

Furthermore, the present invention relates to methods for producing a foreign protein, which comprise the steps of culturing the above-mentioned transformants and isolating a foreign protein from the obtained culture.

To express a foreign protein that one desires to express in microorganisms of the genus *Kocuria* using the present invention's shuttle vector for inducible expression in microorganisms of the genus *Kocuria,* it is necessary to produce a recombinant vector into which a DNA encoding the foreign protein is inserted into a vector of the present invention.

Recombinant vectors of the present invention are produced by inserting a DNA encoding a foreign protein into the vectors of the present invention (for example, pKV15358-2 or pKV15358-3).

In the present invention, the foreign protein that one desires to express is not particularly limited, and includes enzymes, hormones, cytokines, and prepared proteins. Preferred examples of the foreign protein of the present invention include a catalytic enzyme used when producing substances in non-aqueous systems (for example, in organic solvents). An example of the catalytic enzyme includes nitrilase and the vector into which the foreign protein is introduced includes pKV15358-4 (SEQ ID NO: 20).

A transformant of the present invention can be produced by transforming a host with a vector of the present invention or a recombinant vector of the present invention. For example, the electroporation method, calcium phosphate method, and DEAE dextran method can be used as the method for transformation. As the host for expressing a foreign protein or host to be used for propagation and collection of vector plasmids, microorganisms of the genus *Kocuria* can be used.

The present invention enables production of a foreign protein by culturing the above-described transformant and isolating the foreign protein from the obtained culture. As the method for culturing the transformant, one can appropriately select a method suitable for the microorganism of the genus *Kocuria* used as the host. In the method, culturing can be carried out in a non-aqueous system (for example, in an organic solvent).

Expression of a foreign protein can be induced by the vector of the present invention using inducers (for example, ε-caprolactam and isovaleronitrile).

In the present invention, "culture" refers to a material that can be obtained by culturing such as bacterial cells, culture, cell-free extract, and cell membrane. Cell-free extracts can be obtained, for example, by physically disrupting cultured bacterial cells using a homogenizer after adding a sodium phosphate buffer, then separating by centrifugation and collecting the supernatant so that undisrupted bacterial cells (cells) are not present. Cell membrane can be obtained by suspending the pellet obtained by the above-mentioned centrifugation in a solubilizing buffer.

For a foreign protein of interest, a culture solution may be used as it is. Alternatively, a foreign protein of interest may be concentrated and purified using known methods such as dialysis and ammonium sulfate precipitation, or known methods such as various chromatographic methods such as gel filtration, ion exchange, and affinity chromatography alone or in combination.

Furthermore, the present invention relates to a method for producing a substance of interest, wherein the above-mentioned transformant is used as a biological catalyst for producing the substance of interest in a non-aqueous reaction site.

Non-aqueous system refers to a reaction site that is not 100% water, a reaction site having an arbitrary ratio of organic solvent and water, a reaction site which is a supercritical gas, or a reaction site which is an ionic liquid. Microorganisms of the genus *Kocuria* are microorganisms that maintain their cellular structure even in a non-aqueous system. Even when a chemical substance serving as a starting material of the substance of interest is contacted with a transformant of a microorganism of the genus *Kocuria* (used as a biocatalyst) in a non-aqueous reaction site, activity is not lost due to dissolution of the transformant and the substance of interest can be obtained with high productivity. In the present invention, the substance of interest is not particularly limited, and examples include mandelic acid and such.

All prior art documents cited in this specification are incorporated herein by reference.

### Examples

### [Example 1] Estimation of upstream and downstream sequences of the SD sequence suitable for the Kocuria rhizophila NBRC 103217 strain

The entire nucleotide sequence (2.7 Mb) of the *Kocuria rhizophila* NBRC 103217 strain genome was determined, and 2,356 ORFs were estimated from that nucleotide sequence.

Based on the ORF information, the upstream and downstream sequences of the SD sequence that are most suitable for translation were predicted.

Five nucleotides upstream and nine nucleotides downstream of each of the start codons for all ORFs (2,356 ORFs) predicted from the whole genome sequence (2,697,540 bp) of the *Kocuria rhizophila* NBRC 103217 strain were analyzed by quantification theory type 2. In the analysis, as pseudo start codon data, codons ATG, GTG, and TTG which do not overlap with start codons were extracted with a frequency equivalent to that of the start codons.

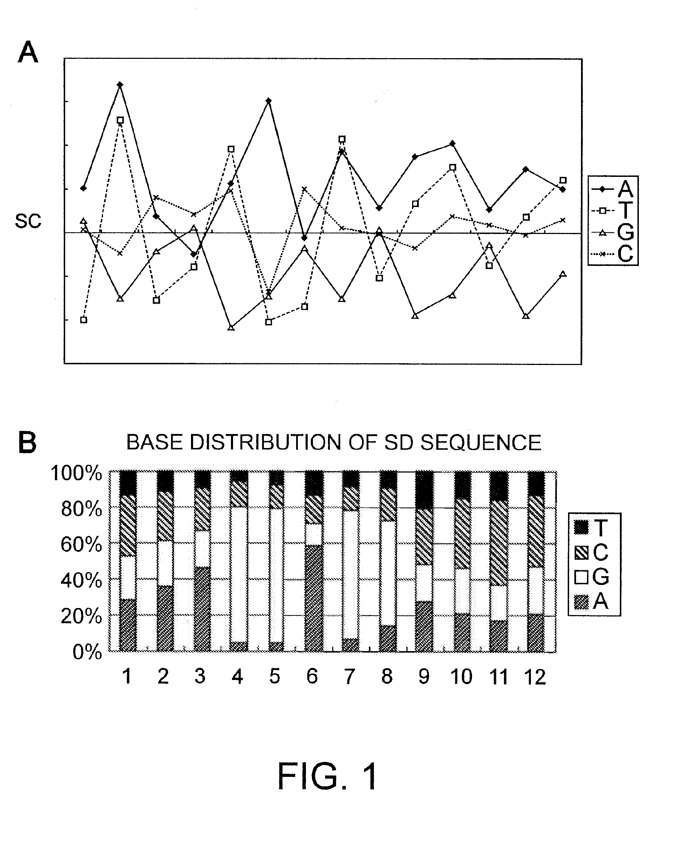

Since the category scores (Fig. 1) indicate importance of each nucleotide, "AACCT" (SEQ ID NO: 8), which is a sequence of nucleotides showing high scores at each position, was predicted to be the most suitable sequence immediately upstream of the start codon for translation.

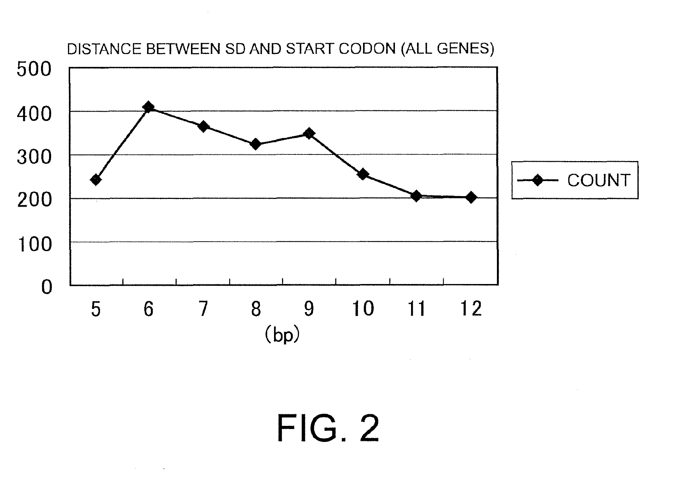

Furthermore, since the sequence of the 3' end of 16S rRNA is AUCACCUCCUUUCU-3' (SEQ ID NO: 9), the SD sequence was predicted to be AGGAGG (SEQ ID NO: 10). As shown in Fig. 2, as a result of analyzing the distribution of the predicted sequences upstream and downstream sequences of the SD sequence in all ORFs of the NBRC 103217 strain, this also confirmed that there is a strong complementarity to the 3' end sequence of the 16S ribosomal RNA (nucleotide sequence of positions 3 to 7). The most frequent number of nucleotides in the sequence between the SD sequence and the start codon was 6 bp, but since 7 bp showed nearly the same value and 7 bp is often optimal in other microorganisms, 7 bp may be the optimal number of nucleotides between the SD sequence and the start codon.

Therefore, 5'-CTAGCAAGGAGGCCAACCT-3' (SEQ ID NO: 11) was predicted as the optimal upstream and downstream sequences of the SD sequence by combining the "AACCT" sequence (SEQ ID NO: 8) upstream of the start codon estimated from quantification analysis type 2 with the results of distribution analysis of the upstream and downstream sequences of the SD sequence.

When introducing into an actual vector, an *Nde*I site which contains an ATG start codon was added to improve convenience at the time of gene introduction, and this was used as the final upstream and downstream sequences of the SD sequence
(5'-CTAGCAAGGAGGCCAACATATG-3', SEQ ID NO: 12).

Next, a transformation system of the *Kocuria rhizophila* NBRC 103217 strain was established using the EZ-TN5<KAN-2>Tnp transposon. It was found out that the efficiency of transformation is greatly improved by using the NBRC 10317 strain bacterial cells grown until OD600 = 0.7 in an LB medium containing 1.5% glycine as the competent cells, and after incubation at 35°C for five minutes, they are used for electroporation. Furthermore, when a transposon containing not only a kanamycin resistance gene which is a selection marker but also the GFP gene was constructed and the NBRC 103217 strain was transformed, a transformant carrying the GFP gene could be obtained even though the transformation efficiency was 3% of that when a transposon carries only the kanamycin resistance.

### [Example 2] Introduction of a plasmid extracted from the Kocuria varians NBRC 15358 strain into the NBRC 103217 strain

Next, a plasmid vector that is replicable in the *Kocuria rhizophila* NBRC 103217 strain was prepared. Since the rescue plasmid (SEQ ID NO: 5) of the plasmid (pKV15358-1) derived from *Kocuria varians* NBRC 15358 is replicable in the *Kocuria rhizophila* NBRC 103217 strain, this plasmid was miniaturized and the upstream and downstream sequences of the SD sequence (SEQ ID NO: 2) found in Example 1 was further added to construct vector pKV15358-3 (4.3 kb) (SEQ ID NO: 7) for the *Kocuria rhizophila* NBRC 103217 strain.

More specifically, the *Kocuria varians* NBRC15358 strain was cultured while shaking at 150 rpm in a 500-mL baffled conical flask containing 100 mL of LB medium at 30°C until the turbidity at 600 nm reached 8.0. Two-hundred milliliters of the culture solution was centrifuged (9,970 x g, 4°C, ten minutes) to collect the cells, and after washing with 15 mL of STE (10 mM Tris-HCl, 0.5 mM EDTA, 5 mM NaCl; pH 8.0), the cells were collected by centrifugation (9,970 x g, 4°C, ten minutes). From the obtained bacterial cells, plasmid DNA was extracted using the Qiagen Plasmid Maxi Kit (Qiagen). When using this kit, 4 mg/mL of lysozyme was added to Buffer P1, and after suspending the bacterial cells in this solution, this was incubated at 37°C for two hours. Operations thereafter were carried out according to the method recommended by the manufacturer.

25 mM MgCl₂ was added to the obtained DNA solution (final 2 mM MgCl₂), and then RingMaster Nuclease (Novagen) was added to achieve 0.5 U/µL. The contaminating chromosomal DNAs were removed by one hour reaction at 37°C. After completion of the reaction, the enzyme was inactivated by incubation at 75°C for ten minutes. Plasmid DNA was collected by ethanol precipitation, and dissolved in 20 µL of TE buffer (10 mM Tris-HCl, 1 mM EDTA; pH 8.0).

To the obtained plasmid, a transposon was inserted according to the instructions for the EZ-Tn5 <R6Kγori / KAN-2> Insertion Kit (Epicentre). TransforMax EC100D pir Electrocompetent *E. coli* (Epicentre) was transformed using the reaction solution, and the transformants were selected on an LB agar plate containing 50 µg/mL kanamycin.

From the six strains of obtained transformants, plasmid DNAs were prepared and the NBRC 103217 strain was transformed. Competent cells of the NBRC 103217 strain were prepared using bacterial cells obtained by growing this bacterial strain in shake culture at 30°C in a 500-mL baffled conical flask containing 100 mL of LB medium supplemented with 1.5% (w/v) glycine until the turbidity at 600 nm reached 0.7. The culture solution was centrifuged (1,400 x g, 4°C, ten minutes) to collect the bacterial cells, and the cells were washed twice with 200 mL and once with 35 mL of cooled sterilized water. The cells were further washed twice with 35 mL of 10% (v/v) glycerol solution, and suspended in 10% (v/v) glycerol solution so that the turbidity at 600 nm becomes approximately 50. The obtained bacterial cell suspension was dispensed into a 1.5 mL tube (100 µL/tube), and after freezing using liquid nitrogen, this was stored at -80°C.

The competent cells were thawed on ice, 0.5 µg of DNA was added, and the NBRC 103217 strain was transformed using the electroporation method (800 Ω, 25 µF, 12.5 kV/cm) using Gene Pulser (Bio-rad). After electroporation and addition of 0.9 mL SOC medium, it was cultured while shaking (30°C, 150 rpm, 16 hours). Then, colonies obtained by 30°C culturing on an SOB agar plate containing 400 µg/mL kanamycin were selected.

Of the six types of plasmids tested for introduction into the NBRC 103217 strain, the plasmid of interest was retained in the cases where four types of plasmids were used.

### [Example 3] Introduction of the Arthrobacter sp. F73 strain-derived nitrilase gene into pKV15358-3

Using the pKV15358-3 vector constructed in Example 2, a nitrilase gene derived from the *Arthrobacter* sp. F73 strain was expressed in the *Kocuria rhizophila* NBRC 103217 strain.

To enable amplification of a DNA fragment with *Nde*I site added to the 5' end and *Xba*I site added to the 3' end of the nitrilase gene derived from *Arthrobacter* sp. F73 strain, two types of primers, F73Nit-309F-NdeI primer
(5'-GTGCATATGAGCGAGTACACCCAGCAATACCGCGTC-3', SEQ ID NO: 13) and F73Nit-1430R-XbaI primer (5'-TCCTCTAGATCAGAGCTGAACGGCCTCCTCCGAACGC-3', SEQ ID NO: 14) were designed. PCR was performed by adding 37.5 U/mL of *Pfu* turbo DNA polymerase to a reaction system containing 0.2 µM of each primer, 1 ng/µL of chromosomal DNA of the F-73 strain as a template, and 2.5% (v/v) DMSO. The PCR included incubation at 98°C for two minutes and thirty seconds, and then 30 cycles of a reaction consisting of three steps of 98°C for 30 seconds, 55°C for one minute, and 72°C for one minute and thirty seconds, followed by incubation at 72°C for ten minutes. The amplified DNA fragment was purified using the GFX PCR Purification Kit (GE Healthcare), and then digested with *Nde*I and *Xba*I, and ligated with plasmid pKV15358-3 digested with the same restriction enzymes. TransforMax EC100D pir Electrocompetent *E. coli* (Epicentre) was transformed with the obtained reaction solution, and transformants carrying the target pKV15358-4 plasmid were obtained from the strains grown in an LB medium containing 50 µg/mL kanamycin.

The NBRC 103217 strain was transformed using plasmid DNA extracted from these transformants to obtain NBRC 103217 strain transformants carrying these plasmids. For bacterial cells obtained by growing the strain in shake culture (250 rpm) at 20°C, 25°C, or 30°C in an LB medium containing 300 µg/mL kanamycin, nitrilase activity was investigated by using mandelonitrile present in the cells as a substrate.

As a result, activity was not seen when grown at 30°C, but activity was observed when grown at 20°C and 25°C. The highest activity was observed when cultured at 25°C for 64 hours. For each bacterial cell, this was approximately 1% of that in *E. coli* (the activity of the NBRC 103217 strain bacterial cell suspension per OD₆₀₀ = 1 was 6.1 mU/mL).

### [Example 4] Production of mandelic acid in a biphasic reaction site using the Kocuria varians NBRC 103217 strain

When the same transformant was used in an ethyl acetate-water biphasic reaction in which mandelic acid production is not observed in *E. coli,* even in a system containing 60% ethyl acetate, it showed a 60% activity of that in an aqueous phase reaction. More specifically, the following examinations were carried out.

The NBRC 103217 strain carrying pKV15358-5 (SEQ ID NO: 21) was cultured while shaking (150 rpm) for 144 hours in 100 mL of LB medium containing 300 µg/mL kanamycin (500-mL baffled conical flask), and the obtained culture solution (OD₆₀₀ = 12.6) was centrifuged (2,300 x g, 4°C, ten minutes) to collect bacterial cells. Bacterial cells were suspended in 50 mM potassium phosphate buffer (pH 8.0), and bacterial cells were reacted with mandelonitrile as the substrate.

The total volume (0.5 mL) of the reaction solution was prepared at 50% (v/v) ethyl acetate, 1 M mandelonitrile, and 50 mM potassium phosphate buffer (pH 8.0), and the bacterial cells were added so that OD₆₀₀ = 10. The reaction was initiated by adding the bacterial cell suspension. Using a BioShaker M·BR-022UP (Taitec), the reaction solution was incubated at 30°C for two hours while shaking at 1,400 rpm. Then, the reaction system was homogenized by adding 0.5 mL of methanol, and centrifuged (20,400 x g, 4°C, ten minutes) to remove bacterial cells. The amount of mandelic acid contained in the obtained supernatant was quantified by HPLC. For the apparatus, LC-10ADvp (SHIMADZU) was used, and for the column, the Inertsil ODS-3 column (φ 4.6 x 150 mm, GL Sciences) was used. Analyses were carried out by setting the column temperature to 40°C, and passing 10 mM phosphoric acid-acetonitrile (55:45) as the eluent at a flow rate of 1 mL/min. The standard curve was produced using 0.02, 1, 5, 10, 15, 20, and 50 mM R-mandelic acid. The retention time was 2.2 minutes. The samples after completion of the bacterial cell reaction were diluted ten-fold using the eluent and subjected to HPLC. In the reaction using the *K. rhizophila* NBRC 103217 (pKV15358-5) bacterial cells in a solvent system of water/ethyl acetate = 1/1, 83.7 g/L of mandelic acid accumulated.

### [Example 5] Searching a promoter highly expressed in the Kocuria rhizophila NBRC 103217 strain

Furthermore, to obtain a promoter for increasing the expression level of a foreign gene, superoxide dismutase was discovered as one of the proteins highly produced in the stationary phase of culture. It was revealed that expression of the nitrilase gene was increased 150 times when a promoter region that expresses the superoxide dismutase-encoding gene was identified, cloned, and introduced into a vector for the *Kocuria rhizophila* NBRC 103217 strain. More specifically, the following examinations were carried out.

The *Kocuria rhizophila* NBRC 103217 strain was cultured while shaking at 150 rpm at 30°C in a 500-mL baffled conical flask containing 100 mL of LB medium until the turbidity at 600 nm reached 11 (65 hours). 20 mL of the culture solution was centrifuged (2,300 x g, 4°C, ten minutes) to collect bacterial cells, the cells were washed with 5 mL of STE (10 mM Tris-HCl, 0.5 mM EDTA, 5 mM NaCl; pH 8.0), and then centrifuged (2,300 x g, 4°C, ten minutes) to collect the cells. The obtained bacterial cells were stored at -30°C.

Bacterial cells in 10 mL of culture solution were suspended in 0.7 mL of solubilizing buffer (10 mM Tris-HCl, 8 M urea, 4% (w/v) CHAPS, 60 mM DTT; pH 7.4), transferred to a 2-mL tube containing 0.5 mL of glass beads (φ 0.1 mm), and then the cells were disrupted using a Multi-Beads shocker (Yasui Kikai) (2,500 rpm, 4°C, 20 minutes). Supernatant obtained from centrifugation (20,400 x g, 4°C, ten minutes) was collected. Protein was purified from 100 µL of the obtained supernatant using the ReadyPrep 2-D Cleanup Kit (Bio-rad). The obtained protein was dissolved in 60 µL of swelling buffer (7 M urea, 2 M thiourea, 2% (w/v) CHAPS, 20 mM DTT, 0.2% (v/v) Biolyte 3-10, 0.039% (w/v) tris-(2-cyanoethyl)phosphine) to produce a protein solution for two-dimensional electrophoresis.

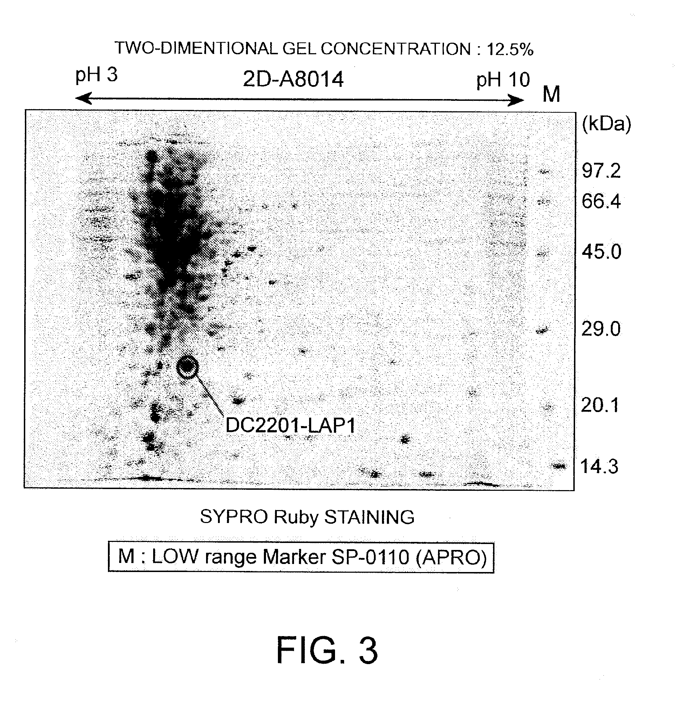

Two-dimensional electrophoresis was carried out by subjecting 50 µg of the protein to isoelectric focusing using PROTEAN IEF Cell (Bio-rad). IPG ReadyStrip pH 3-10NL (7 cm, Bio-rad) was used for the isoelectric focusing electrophoresis gel. After the electrophoresis, the isoelectric focusing gel was removed, and this was subjected to SDS-PAGE using a 12.5% uniform gel. Thereafter, the gel was subjected to protein staining using SYPRO Ruby (Invitrogen), and then a large spot which appeared to be NBRC 103217-LAP1 was excised (Fig. 3).

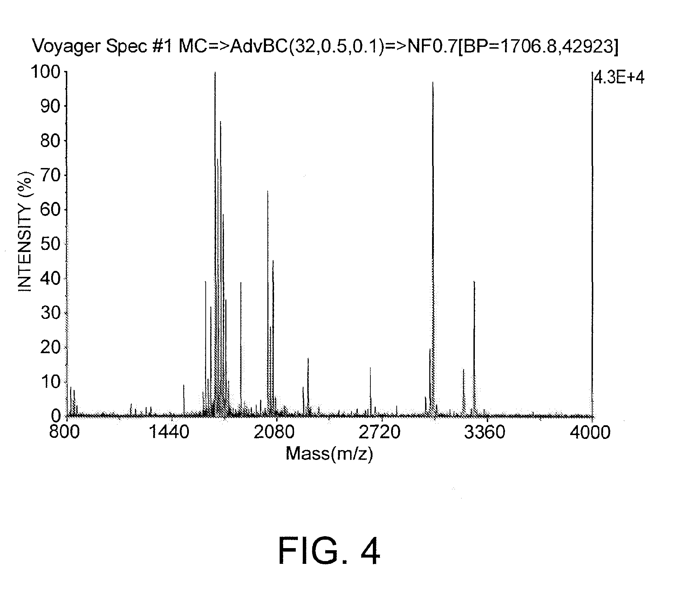

Tris-HCl (pH 8.0) containing trypsin was added to the excised gel slice, and this was incubated at 35°C for 20 hours. After the obtained reaction solution was desalted using ZipTip C18 (Millipore), this was mixed with a matrix (α-cyano-4-hydroxyxinnamic acid solution), and spotted onto a plate. After air drying, this was subjected to MALDI-TOF MS. Using Voyager-DE STR (Applied Biosystems) in the reflector mode, peptides with cationic m/z values of 800 to 4,000 were detected (Fig. 4). Among proteins encoded by the ORFs of the NBRC 103217 strain, search for a protein predicted to be NBRC 103217-LAP1 was carried out using Mascot (Matrix science) based on the monoisotopic mass of the obtained peptides, and it is highly possible that the protein was KRH_00400 encoding superoxide dismutase (SodA).

Therefore, the superoxide dismutase gene sodA (KRH_00400) was found to be highly expressed in the NBRC 103217 strain. Accordingly, when searching for a promoter predicted upstream of this gene, sequences "TTGACC" (SEQ ID NO: 15) and "GACAGT" (SEQ ID NO: 16) showing similarity to the -35 region and -10 region of an *E. coli*-derived promoter were found 67 bp to 62 bp and 43 bp to 38 bp upstream of the sodA gene start codon, respectively. To amplify DNA fragments containing these promoter regions by PCR, gene0045-F1 primer (5'-GGCGTCGACCGAGGGTAGGGCGGGAGTG-3', SEQ ID NO: 17), gene0045-R1 primer (5'-TCGACTAGTGGCGTGATGGTTCAACTGTC-3', SEQ ID NO: 18), and gene0045-R2-v2 primer (5'-AGCCATATGGAAACATCCTTTCGTTTGCG-3', SEQ ID NO: 19) were prepared. Using 1 ng/µL of chromosomal DNA of the NBRC 103217 strain as the template, and either the gene0045-F1 and gene0045-R1 primer pair or the gene0045-F1 and gene0045-R2-v2 primer pair (0.2 µM each), PCR was performed by adding 25 U/mL of PrimeSTAR DNA Polymerase. The PCR was performed by incubation at 96°C for five minutes, and then 30 cycles of a reaction consisting of three steps of: 98°C for 10 seconds; 55°C for 15 seconds; and 72°C for thirty seconds, followed by incubation at 72°C for ten minutes. The obtained DNA fragments (PsodA1 and PsodA2) were purified using the GFX PCR Purification Kit (GE Healthcare), and then they were double-digested with *Sal*I-*Spe*I and *Nde*I-*Sal*I, respectively. The obtained DNA fragments were ligated with fragments each produced by digesting pKV15358-8 (SEQ ID NO: 24), which is a vector for promoter evaluation, with the same restriction enzymes. *E. coli* JM109 was transformed using the reaction solution, and transformants carrying the plasmids of interest, pKV15358-5 (SEQ ID NO: 21) and pKV15358-6 (SEQ ID NO: 22), were obtained from the strains grown in an LB medium containing 50 µg/mL kanamycin. Using plasmid DNAs extracted from the obtained transformants, the NBRC 103217 strain was transformed, and by culturing at 30°C on an SOB agar plate containing 400 µg/mL kanamycin, the NBRC 103217 strain transformants carrying each of the plasmids were obtained.

When each of the obtained transformants were cultured while shaking (150 rpm) in 100 mL of an LB medium without kanamycin and an LB medium containing 300 µg/mL kanamycin (500-mL baffled conical flask), the NBRC 103217 strain transformant that carries pKV15358-5 with optimized upstream and downstream sequences of the SD sequences showed a nitrilase activity 86 times greater than that of the NBRC 103217 strain transformant carrying pKV15358-4 which does not contain an sodA-derived promoter (P_{sodA}). Furthermore, in the NBRC 10317 strain, the transformant exhibited an activity three times greater than that of pKV15358-6, to which sodA's original upstream and downstream sequences of the SD sequence was added.

To produce high-expression vectors for the NBRC 103217 strain, shuttle vectors of the NBRC 103217 strain and *E. coli,* which are the *Bsp*HI-*Nde*I fragment (2,309 bp) of pKV15358-9 (SEQ ID NO: 25) and the *Bsp*HI-*Nde*I fragment (1,253 bp) of pKV15358-5, were ligated. The reaction solutions were used to transform *E. coli* JM109, and from the strains grown in an LB medium containing 50 µg/mL kanamycin, the plasmids of interest pKV15358-5 and pKV15358-7 (SEQ ID NO: 23) were obtained as high-expression vectors for the NBRC 103217 strain.

### Industrial Applicability

By introducing shuttle vectors developed by the present inventors into microorganisms belonging to the genus *Kocuria* which can maintain their cellular structure in non-aqueous systems, expression of a foreign protein of interest can be induced in non-aqueous systems. Furthermore, by using microorganisms introduced with a shuttle vector of the present invention, the activity of a foreign protein (for example, catalyst activity) can be expressed in a non-aqueous system.

## Claims

1. A vector capable of expressing a foreign protein in a microorganism, which comprises a polynucleotide comprising the nucleotide sequence of SEQ ID NO: 1, or a polynucleotide that hybridizes under stringent conditions with a complementary strand of a polynucleotide comprising the nucleotide sequence of SEQ ID NO: 1.

2. The vector of claim 1, wherein the vector additionally comprises a polynucleotide comprising the nucleotide sequence of SEQ ID NO: 2, or a polynucleotide that hybridizes under stringent conditions with a complementary strand of a polynucleotide comprising the nucleotide sequence of SEQ ID NO: 2.

3. The vector of claim 1 or 2, wherein the vector additionally comprises a polynucleotide comprising the nucleotide sequence of SEQ ID NO: 3 or 4, or a polynucleotide that hybridizes under stringent conditions with a complementary strand of a polynucleotide comprising the nucleotide sequence of SEQ ID NO: 3 or 4.

4. The vector of any one of claims 1 to 3, wherein the microorganism in which a foreign protein is expressed is a microorganism belonging to the genus *Kocuria,* the genus *Micrococcus,* or the genus *Arthrobacter.*

5. The vector of claim 4, wherein the microorganism belonging to the genus *Kocuria* is the *Kocuria rhizophila* NBRC 103217 strain.

6. A recombinant vector in which a DNA encoding a foreign protein is inserted into the vector of any one of claims 1 to 5.

7. A transformant in which a host is transformed with the vector of any one of claims 1 to 5 and the recombinant vector of claim 6.

8. A method for producing a foreign protein, which comprises the steps of culturing the transformant of claim 7 and isolating the foreign protein from the obtained culture.

9. A method for producing a substance of interest, wherein the transformant of claim 7 is used as a biocatalyst for producing the substance of interest in a non-aqueous reaction site.
